# EUROPEAN PATENT APPLICATION

(11) **EP 4 324 832 A2**
(43) Date of publication of application: **21.02.2024**
(21) Application number: 23215916.0
(22) Date of filing: 22.09.2021
(51) Int. Cl.: C07D 417/14

(54) **IMMUNOMODULATORY COMPOUNDS AND USE THEREOF FOR THE TREATMENT AND/OR PREVENTION OF INFECTIOUS DISEASES**

(30) Priority: 23.09.2020 EP 20197867
(62) Divisional of application: 21782527.2
(71) Applicant: AC BioScience SA, 1066 Epalinges (CH)
(72) Inventor: AUCLAIR, Christian, 78730 Saint-Arnoult en Yvelines (FR); IVES, Annette, 1066 Epalinges (CH)
(74) Representative: KATZAROV S.A.

(57) **Abstract**

The invention provides immunomodulatory compounds, adjuvant compositions comprising thereof, immunogenic compositions comprising thereof and uses of immunomodulatory compounds as adjuvants in vaccination, for increasing the immune response via the improvement of maturation and activation of dendritic cells, and for the treatment and/or prevention of infectious diseases, cancer and autoimmune diseases.

## Description

### FIELD OF THE INVENTION

The invention provides immunomodulatory compounds, adjuvant compositions comprising thereof, immunogenic compositions comprising thereof and uses of immunomodulatory compounds as adjuvants in vaccination, for increasing the immune response via the improvement of maturation and activation of dendritic cells, and for the treatment and/or prevention of infectious diseases, cancer and autoimmune diseases.

### BACKGROUND OF THE INVENTION

The innate and adaptive immune systems work in concert to protect humans from infection and or other disease-causing agents, which can include transformed cells in the case of cancer. In humans, the innate immune system is rapidly activated through the recognition of evolutionarily conserved motifs called damage or pathogen-associated molecular patterns (DAMPS and PAMPS) to produce small chemical and protein mediators (such as cytokines, chemokines, reactive nitrogen species and oxygen species) and other defensive systems including the complement cascade. Yet, this system provides no immunological memory and means that every subsequent challenge takes the same time to respond, and in the case of infections, clear the insulting pathogen. The human adaptive immune system relies on the activation of T and B lymphocytes that recognize specific peptide sequences (Antigens) derived from proteins of pathogens, motifs derived from cancerous cells (tumor-associated Antigens) or "self' antigens presented in the context of autoimmunity. These T cells, following activation, rapidly proliferate into a large number of short-lived blasts, while a small percentage differentiate into long-lived memory T cells that can rapidly be reactivated for protection from clinical pathology in a subsequent challenge.

Antigen-presenting cells provide the bridge between the innate and adaptive immune systems, enabling potent protective immune response generation. Of the cells of the immune system, the main "professional antigen-presenting cell" is the dendritic cell, yet under certain circumstances other cells can provide this function including macrophages, monocytes, B lymphocytes or inflammation-activated epithelial cells. Within the immune system there are different types of dendritic cells characterized by their immunostimulatory potential and Cluster of differentiation (CD) expression profile and can be of plasmocytoid or myeloid in origin. These cells all share the capacity to present antigens after intracellular processing through antigen loading, transport and cell surface expression Major Histocompatibility Complex (MHC) I and/or II: antigen complexes. All cells express MHC-I, yet MHC-II is predominantly found on antigen presenting cells. MHC-II dependent antigen presentation gives rise to CD4+ T helper cell dependent responses while MHC-I present antigens in a process called Cross Presentation, resulting in the activation of cytotoxic CD8+ T cells which are important for anti-viral and anti-tumor immune responses.

### Antigen uptake and processing

The first step in antigen presentation is the uptake of endogenous and exogenous antigens that are either particulate or soluble in nature. This occurs either via phagocytosis or endocytosis. Subsequent to internalization, proteins and long peptides are processed via proteolysis into small peptide fragments either in the cytosol by the immunoproteosome or an endosomallydependent mechanism reliant on the formation of acidified phagolysosomes and cathepsinmediated cleavage into short peptides.

In general, MHC-I:peptide complex formation uses cytosolic processing and antigen loading within the Endoplasmic reticulum through the formation of the Peptide-Loading Complex comprised of the MHC-I heavy chain B2-microglobulin, Transporter associated with antigen processing (TAP1,2) Tapasin calreticulin and ERp57.These complexes are then transferred via the golgi body to the cell surface.

MHC-II:peptide complex formation predominantly uses the endosomal pathway, whereby MHC-II complexes are translocated from the ER in complex with its chaperone protein CD74 to late endosomes where it is processed causing dissociation to enable peptide binding. Once MHC:peptide complexes are formed less well understood mechanisms mediate its translocation to the cell surface. For both MHC-I and MHC-II dependent protein and long peptide processing results in the generation of peptidic immune responsive epitopes of between 8-11 or 13-17 amino acids in length for MHC-I and MHC-II:peptide complex formation, respectively.

### Antigen presentation and adaptive immune responses

Dendritic cells, through phagocytosis, can internalize microbes (including bacteria, parasites, fungi, and viruses) but they can also uptake necrotic and apoptotic cells in specific receptor mediated processes. Activation of receptors (including that of Toll Like Receptors-TLR) induces innate immune response activation, cytokine production and the expression of costimulatory molecules that work in concert with MHC:peptide complexes and polarizing cytokines for lymphocyte (T cell) activation.

The immunological synapse is responsible for the presentation of Ags and is a specialized junction involving cellular polarization and interaction between antigen presenting cells and naive CD4+ T lymphocytes. For T cell activation, 3 discrete signals need to occur. Signal 1 as mentioned previously is the formation of MHC:peptide complexes on the cell surface that interact with the T cell receptor (TCR) and activation of dendritic cells improves the quantity, stability and longevity of these complexes on the cell surface to facilitate interaction with CD4+ T cells that are capable of recognizing the specific peptide sequence (or epitope). Signals 2 and 3 are provided by costimulatory or co-inhibitory molecules including CD80, CD86 and CD40, and/or programmed death-1 ligands (PD-L1 and PD-L2), that interact with their respective ligands on the CD4+ T cell of CD28, CD40L and PD-1, and secreted cytokines, respectively. These can be upregulated through activation of the innate immune system through ligation with evolutionarily conserved pathogen or damage-associated molecular patterns (PAMPS or DAMPS) with extracellular or intracellular receptors. Co-inhibitory molecules are negative regulators of this process. The subtype of Dcs directly impact the type of adaptive immune response generated. Signal 2 and 3 are essential in determining the should the immune system respond, to what extent and in the polarization of the adaptive immune system towards Tfh (FollicularT helper cells), Th1, Th2, Th9 or Th17 polarized responses in CD4+ T cells. Alternatively, Dc and naive T cell interaction can lead to regulatory responses given by IL-10 and TGF-beta or the presentation peptide:MHC complexes in the absence of signal 2 or 3. Th1 or cell-mediated responses are IL12p70, IFN type I and IFN-y dependent, produced by the APC and the T cell, respectively, and are effective against intracellular pathogens and eliminating cancerous cells . Th2 responses are characterized by the absence of IL12p70 and the generation of IL-4,-5,-13 by naive T cells for humoral, antibody-dependent immune responses targeting extracellular pathogens, via B- cell activation and mast cell degranulation, and can work in concert with Tfh. Th17 responses are involved in inflammation, activation of neutrophils and the fight against extracellular bacteria and mucosal defense. Here T cells produce IL-17, IL17F, IL-6, IL-22 and TNF while DCs polarize these responses through IL23 or a combination of IL-6 and TGF-Beta.

Importantly, Cross presentation to CD8+ T cells is most potent in IL-12 producing DC. Here activation and/or maturation of this cell type improves the stabilization of MHC-I:peptide complexes on the cell surface mediated, in part by NADPHoxidase activity or NOD-Like receptor caspase recruitment domain-containing protein (NLRC5) dependent H2-Kb transcription induction.

Upon interaction with APC, naive T cells that express the T cell receptor capable of recognizing the presented antigen rapidly proliferate and induce cytokine production according to the polarizing signals received. These T cells undergo clonal expansion to generate a high number of blasts that are short-lived effector cells, while a smaller percentage differentiate into memory T cells that are long-lived, are retained within the circulation and can rapidly respond in subsequent challenge. In the case of humoral-B-cell dependent-immunity activation occurs in CD4+ T helper cells dependent and independent mechanisms exist where the latter is required for response to complex protein derived antigens. Although B cells can act as an antigen-presenting cell these are predominantly recognizing highly expressed carbohydrate moieties.

The development of potent immune responses and immunological memory are crucial factors for disease resolution, vaccine-induced protection and protection from subsequent challenge. Factors including the ability of the matured antigen-presenting cells to remain sufficiently activated to migrate to the draining lymph node for instruction to T and B cell immune responses, presentation of sufficient amount of MHC:peptide antigen complexes in the appropriate context to induce expansion of lymphocytes into antigen-specific effector cells, generate memory B and T cells while reducing regulatory cell populations.

### Immune subversion mechanism of pathogens

Given that human immune system is has evolved a highly mechanistic approach to combat disease and provide protection from infection, it is not surprising that many pathogens, including those that are reside within cells, have developed mechanisms to subvert the immune system. These mechanisms include the modulation of cytoskeletal rearrangements, modulation of the endo-phagolysosomal compartment function, inhibition of cytokine/chemokine production through evasion of PAMP and DAMP recognition, impaired responsiveness to microbial killing mechanisms, and modulation of one or multiple aspects of the immunological synapse. In some cases, these microbes act via modulating the ability of dendritic cells, and other antigen presenting cells, in their capability to present antigens in the context of MHC on the cell surface, modulate expression of co-stimulatory molecules, and/or modulate their ability to secrete and/or respond to cytokine/chemokines. Individual mechanisms have evolved for a variety of pathogens, targeting different aspects of the innate and adaptive immune response and is found in successful pathogens of all types including bacteria, viruses, fungi, protozoa and parasites. For example, Mycobacterium tuberculosis, Yersinia spp., and Brucella spp. impair DC maturation, modulate MHC expression co-stimulatory molecule expression, and enhance regulatory responses all of which favor bacterial replication through the production of specific inhibitory moieties. Cross-presentation via MHC-I and activation of cytotoxic T cells, crucial for anti-viral immunity is also impacted. Parasites, as with other pathogens, have evolved evasion mechanisms for the development of Th1 (IFNy and IL12p70) and cytotoxic T cell immune responses needed for disease resolution as well and long-term prevention of reinfection. Leishmania parasites modulate Dc functioning through their "silent" entry into cells with minimal upregulation of MHCI/II, cytokine secretion, and co-stimulatory molecule expression. Additionally, these DC have impaired in DC migratory capacity and impacting trafficking between infection sites and draining lymph nodes. Increased expression of MHC-II and high CD80 and CD86 has previously been demonstrated to prevent apoptosis/anergy of T cells and is involved in the release of IL-2 a known factor for crucial for memory T cells. Furthermore, CD80/86 was essential for controlling mycobacterial infection using CD80/CD86 double knockout mice. Similar results were obtained in HCV, measles virus and parasitic models of infection for CD80 and/or CD86. Notably, both of these co-stimulatory molecules have been associated with the development of cell-mediated (Th1) immune responses.

Immunotherapy has been demonstrated as a relevant strategy for the destruction of cancer cells and is rapidly becoming a first-line treatment strategy in many countries. A similar strategy for infections either alone or in conjunction with anti-microbials would expand the spectrum of diseases capable of being treated and combat the ever-increasing occurrence of resistance and the re/emergence of infections of high public health concern. The additional advantage of such an approach would be to shorten treatment regimens, reduce health system spending, and as the individual determinants of protection given by antigen specific immune responses are patient specific-limit the possibility for escape mutants to arise.

These strategies are very valid, but the development of immunomodulatory compounds is also relevant for the development of either prophylactic or therapeutic vaccines. Vaccines are the most-cost effective and minimally invasive manner through which to treat and/or protect populations from infection. Currently, for cutaneous forms of leishmaniasis, topical therapies are in clinical development that couple the TLR agonist imiquimod with the anti-parasitic agent paromycin.

### Case for vaccination

The development of successful vaccines against disease has led to either the global elimination of several life-threatening diseases such as smallpox or a dramatic reduction in the burden and incidence of many other infectious diseases of viral and bacterial origin.

Current vaccines can be separated into live-attenuated or killed pathogens, subunit vaccines (containing immune-responsive fragments of the pathogen), toxoid vaccines (targeting pathogen secreted toxin that mediate damage) and conjugated vaccines. Vaccination strategies can further be separated into prophylactic (pre-exposure) and therapeutic vaccines (postexposure), the former of which is most sought after to target highly communicable pathogens. Of the vaccines developed to date, most are dependent on antibodies for protection with only a few have demonstrable roles in eliciting T cell-dependent responses and of which these are predominantly of the live-attenuated or heat-killed category.

Antibodies mediate protection through a multitude of ways including neutralizing toxin activity, preventing viral replication and entry into cells, promoting opsonization of pathogens and their recognition by phagocytes, and/or activating the complement cascade. However, in many circumstances these mechanisms are not sufficient to induce protection from clinical disease presentation after exposure or mediate disease resolution. Hence vaccination is still not possible for many diseases. This is the case for many intracellular pathogens including bacteria, viruses and parasites, who themselves elicit immune subversion mechanisms or in the case of therapeutic vaccines for cancer. For these diseases, improvement of CD4-dependent Th1, Th17 and Tfh and/or CD8+-mediated killed mechanisms is thought to be key.

Thus, knowing that activation of the dendritic cells and the resulting innate immune response skews adaptive immune response polarization, considerable effort is being placed into finding novel adjuvants or co-adjuvants formulated with the right route of administration and antigentype (most of which are subunit vaccines). All of which are used to improve the priming phase of naive T cells which is guided by the degree and duration of antigen stimulation by antigen-presenting cells.

Therefore, there is still a need for an immunomodulatory compound, effective in modulating the immune system, enhancing immune response and to be used to efficiently treat infectious diseases.

### SUMMARY OF THE INVENTION

An aspect of the present invention provides an immunomodulatory compound 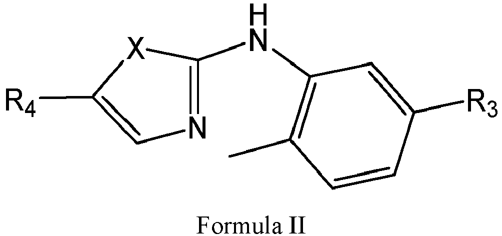 or pharmaceutically acceptable salts thereof
wherein
X is selected from the group comprising NH, O, and S;
R₃ is selected from the group comprising -H, -OH, -OCH₃, -CH₂-OH, and -S-CH₃;
R₄ is selected from the group comprising -CH₃, -CH₂-CH₃,

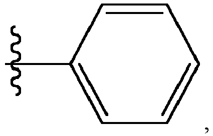

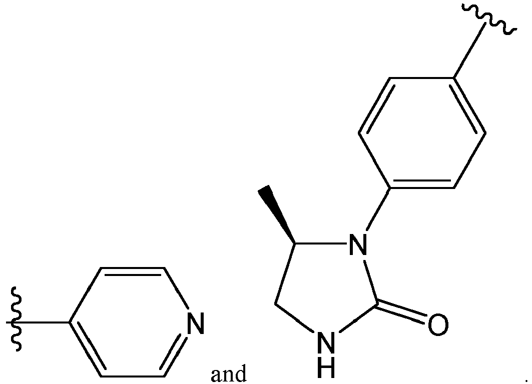

A further aspect of the present invention provides an adjuvant composition comprising the immunomodulatory compound of the invention and at least one pharmaceutically acceptable excipient and/or carrier.

Another aspect of the present invention provides an immunogenic composition comprising an antigen and the immunomodulatory compound of the invention.

Another aspect of the present invention provides an immunomodulatory compound of the invention for use as an adjuvant in vaccination.

Another aspect of the present invention provides an immunomodulatory compound of the invention or an immunogenic composition of the invention for use in the method of improving the immune response in a subject.

Another aspect of the present invention provides an immunogenic composition of the invention or an immunomodulatory compound of the invention for use in the method of treating and/or preventing an infectious disease, wherein the infectious disease is caused by pathogenic microorganisms selected from the group comprising bacteria, viruses, fungi, protozoal pathogens and parasites.

### BRIEF DESCRIPTION OF THE FIGURES

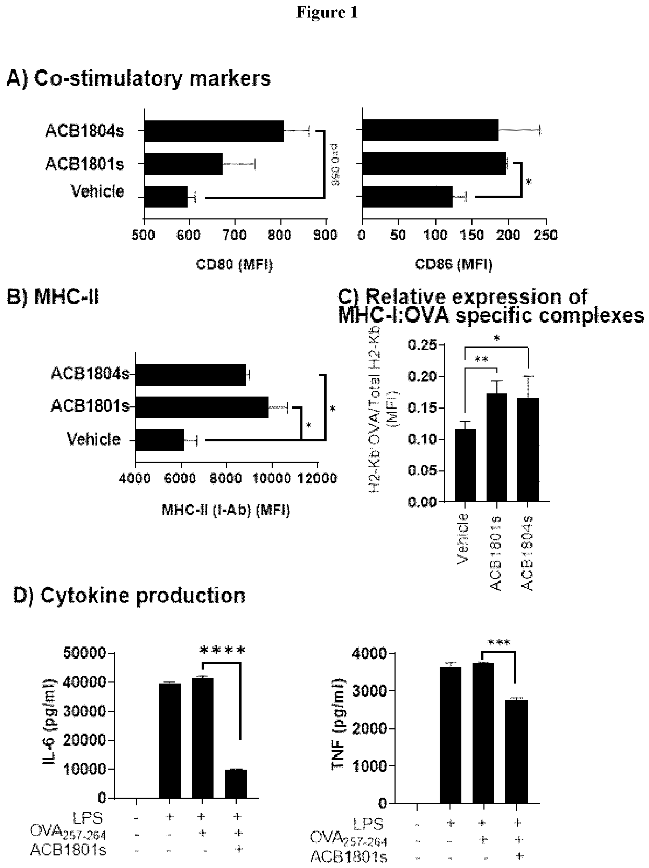

**Figure 1** shows modulation of activation and antigen-presenting capability of Dendritic cells. Bone-marrow derived dendritic cells (BMDC) were differentiated from bone marrow precursor cells of 3 individual female C57BL/6 mice for 7 days in the present of GM-CSF and IL-4 at 250 and 5 UI/ml, respectively. After differentiation cells were pre-treated with ACB1801s (harmine HCl) or ACB1804s (Harmol HCl) for 1 hour prior to stimulation with ultrapure *E. coli* 0111.B5 LPS at 100ng/ml and OVA₂₅₇₋₂₆₄ peptides at 10µM for 24 hours. Cells were analysed for expression of co-stimulatory markers and MHC complexes at the cell surface by FACS, gated on the live CD11c+ population. Panel A) Median fluorescence intensity (MFI) of CD40, CD80 and CD86 expression. Panel B) Overall expression of MHC-II, given by the expression of I-Ab. Panel C) Relative surface expression of MHC-I (H2-Kb): OVA257-264 peptide complexes over the total surface expression of H2-Kb. Panel D) Quantification of cytokine secretion by ELISA. Data is expressed as the mean +/- standard error of the mean (SEM). Statistical significance was determined by a student's t-test with *p≤0.05.

### DETAILED DESCRIPTION OF THE INVENTION

All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components. Also as used in the specification and claims, the language "comprising" can include analogous embodiments described in terms of "consisting of " and/or "consisting essentially of".

As used in the specification and claims, the singular form "a", "an" and "the" include plural references unless the context clearly dictates otherwise.

As used in the specification and claims, the term "and/or" used in a phrase such as "A and/or B" herein is intended to include "A and B", "A or B", "A", and "B".

As used in the specification and claims, the term "at least one" used in a phrase such as "at least one immunomodulatory compound" is intended to include one immunomodulatory compound of the invention or more than one immunomodulatory compound of the invention, such as a combination or two, three or more different immunomodulatory compounds of the invention of Formula I and/or Formula II and/or Formula III and/or Formula IV and/or Formula V and/or Formula VI.

As used herein the terms "subject" and "patient" are well-recognized in the art, and, are used herein to refer to a mammal, and most preferably a human. In some embodiments, the subject is a subject in need of treatment or a subject being infected and/or having an infectious disease, who is likely to benefit from a treatment of the present invention. The term does not denote a particular age or sex. Thus, adult and newborn subjects, whether male or female, are intended to be covered.

As used herein the term "pharmaceutically acceptable excipients and/or carriers" means that the compositions or components thereof so described are suitable for use in contact with a mammal body, preferably human body, or suitable for any other means of administration to human body without undue toxicity, incompatibility, instability, irritability, allergic response, and the like. The term includes any and all solvents, dispersion media, coatings, antibacterial and antifungal agents, isotonic and absorption delaying agents and the like. The use of such media and agents for pharmaceutically active substances is well known in the art. In addition, various adjuvants such as are commonly used in the art may be included. Considerations for the inclusion of various components in pharmaceutical compositions are described, for example, in Gilman et al. (Eds.) (1990); Goodman and Gilman's: The Pharmacological Basis of Therapeutics, 8th Ed., Pergamon Press, which is incorporated herein by reference in its entirety.

The term "treat" and its grammatical variants (for example "to treat," "treating," and "treatment") refer to administration of an active pharmaceutical ingredient or the combination therapy of a present invention to a subject with the purpose of ameliorating or reducing the incidence of one or more symptoms of a condition or disease state in the subject. Such symptoms may be chronic or acute; and such amelioration may be partial or complete. In the present context, treatment entails administering the immunomodulatory compound of the invention or the immunogenic composition of the invention to a subject.

The term "therapeutically effective amount," as used herein, refers to any amount of a specific component or combination of components that will cause a reduction of symptoms, disappearance of the symptoms or relief from symptoms related to for example infectious disease, when applied, either once, or repeatedly over time. For example, a therapeutically effective amount of an immunomodulatory compound of the invention is an amount sufficient to effect beneficial or desired clinical results and/or sufficient to ameliorate, stabilize, reverse, slow and/or delay progression of an infectious disease or to cure an infectious disease. Therapeutically effective amounts, which are non-cytotoxic amounts, can be readily determined by persons skilled in the art using routine experimentation and using tests and measures commonly employed in the art, or can be based upon the subjective response of patients undergoing treatment.

The term "adjuvant" as used herein, means a substance that enhances a subject's immune response to an antigen and/or a substance that enhances subject's immune response and/or treatment efficacy to a drug, a medication or a therapeutic agent (such as for example anticancer agents (preferably cancer immunotherapeutic agents selected from the group comprising immune checkpoint inhibitor, TCR-T cells, CAR-T cells and combinations thereof), antiinfectious agents (preferably antibiotics, anti-bacterial agents, anti-viral agents, antifungal agents, antiparasitic agents, osmotic diuretics, anti-convulsants, anti-pyretics, immunomodulating drugs and combinations thereof). Typically, a substance enhancing the subject's immune response and/or treatment efficacy to a drug, a medication or a therapeutic agent, is administered in combination with such drug, medication or therapeutical agent as an adjuvant therapy given in addition to the primary or initial therapy to maximize its effectiveness). The immunogenic composition of the invention may optionally comprise one or more adjuvants.

The term "antigen" as used herein, refers to a substance or molecule that induces, elicits or triggers an immune response in a cell, tissue or organism. An antigen may originate either from the body, such as cancer antigen, or from the external environment, for instance, from infectious agents. Antigens may be, in whole or part, endogenous or exogenous peptides, proteins or polypeptides of interest or fragments thereof.

The present innovation provides use of the immunomodulatory compounds of the invention in methods through which dendritic cell-mediated activation, other antigen-presenting cell-mediated activation and antigen presentation can be enhanced so as to improve immunological synapse formation/stability to facilitate adaptive immune response generation in T and B lymphocytes. Thereby, improving the development of long-lasting and disease-specific immune responses that facilitate pathogenic microorganism clearance and enhanced vaccine efficiency.

The inventors demonstrated that the immunomodulatory compounds of the invention improve the degree of antigen presentation in the context of MHC and increase costimulatory molecule expression in the absence of TLR stimulation on immature, or previously matured dendritic cells (DC). This effect was pronounced during antigen presentation in the presence of an adjuvant (*E.coli-derived* Lipopolysaccharide(LPS)), while reducing high cytokine levels. Thus, the use of the immunomodulatory compounds of the invention adds to existing vaccine formulations to enhance their efficacy while limiting the known pathological effects of high cytokine levels. Indeed, cytokines are potently generated in vaccines and TLR-ligands are efficient at this process yet the strength of stimulation induced can result in negative feedback signals limiting responsiveness or eliciting adjuvant-induced toxicity. Additionally, soluble TLR-based adjuvants responses may be relatively short-lived which may limit their action in antigen presentation promotion. A detoxified LPS-derivative monophosphorylated Lipid A-MPLA is used as a vaccine adjuvant over LPS from which it was derived as it retains adjuvant efficacy but with limited toxicity. MPLA was 0.1% as toxic as compared to LPS in pre-clinical studies and displays reduced cytokine production (reviewed in Casella et al, 2008, Cell Mol Life Sci. 2008 Oct; 65(20): 3231-3240.).

The use of the TLR-ligand of E.coli-derived Lipopolysaccharide (LPS) serves to not only to illustrate adjuvant action but provides evidence of the capability to modulate immune responses during infection and innate immune stimulation during inflammation and autoimmunity.

In an embodiment, the present invention provides immunomodulatory compounds selected from the group consisting of 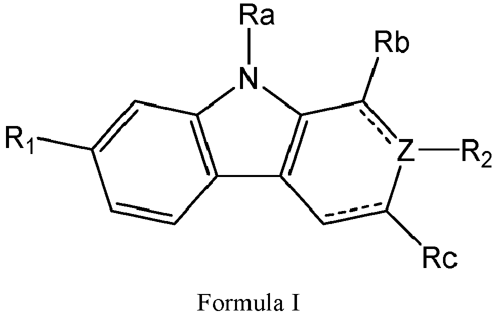 preferably 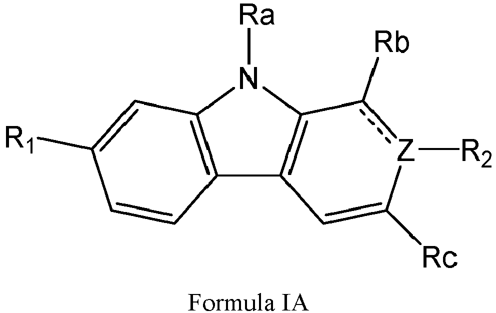 or pharmaceutically acceptable salts thereof
wherein
Z is N or C; preferably Z is N;
R₁ is selected from the group comprising -H, =O, -OH, -S-CH₃, -OCH₃, and -CH₂OH; preferably R₁ is selected from the group comprising -H, =O, -OH, -OCH₃;
R₂ is absent or selected from the group comprising -H, -CH₃, and -CH₂-CH₃; preferably R₂ is absent or is -H;
Ra is selected from the group comprising -H, -CH₃ and -(CH₂)n-CH₃, wherein n is 1 to 20 or 1 to 10, preferably n is 1 to 5 or 1 to 3; preferably Ra is -H;
Rb is -H or -CH₃;
Rc is -H or -NH₂;
Preferably Z is N and R₂ is absent,
When R₁ is =O, then Z is N and R₂ is -H; 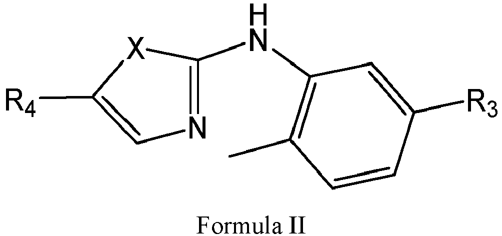 or pharmaceutically acceptable salts thereof
   wherein
   X is selected from the group comprising NH, O, and S;
   R₃ is selected from the group comprising -H, -OH, -OCH₃, -CH₂-OH, and -S-CH₃; preferably R₃ is -OCH₃ or -S-CH₃;
   R₄ is selected from the group comprising -CH₃, -CH₂-CH₃, 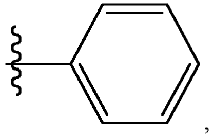 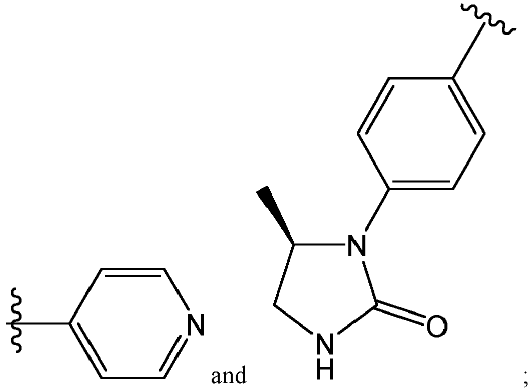 preferably R₄ is -CH₃ or -CH₂-CH₃; 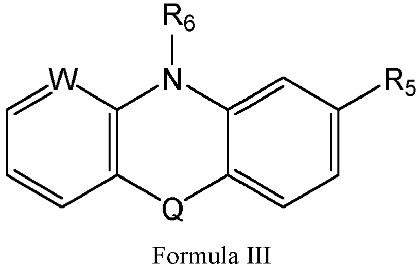 or pharmaceutically acceptable salts thereof
      wherein
      W is N or CH; preferably W is CH;
      Q is selected from the group comprising O, S and NH; preferably Q is S;
      R₅ is selected from the group comprising -H, -OH, -OCH₃, -CH₂OH, and -S-CH₃; preferably R₅ is -H;
      R₆ is selected from the group comprising -H, -CH₃, -CH₂-CH₃, -CH₂-CH₂-N(CH₃)₂, 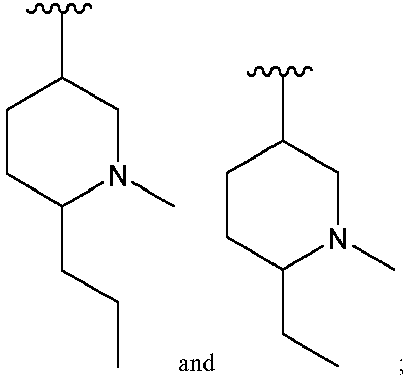 preferably R₆ is -H. 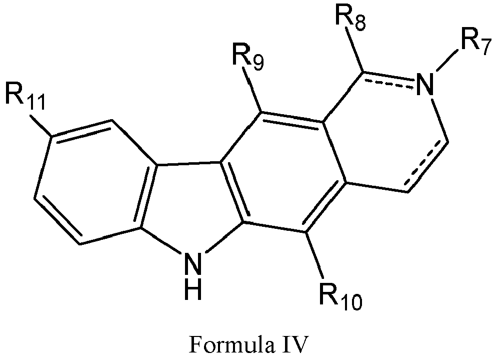 or pharmaceutically acceptable salts thereof
         wherein
         R7 is absent or selected from the group comprising -H, -CH₃ and -CH₂-CH₃;
         R8 is -H or -CH₃;
         R9 is -H or -CH₃; preferably R9 is -CH₃;
         R10 is -H or CH₃; preferably R10 is -CH₃;
         R11 is selected from the group comprising -H, -OH and -O-CH₃; 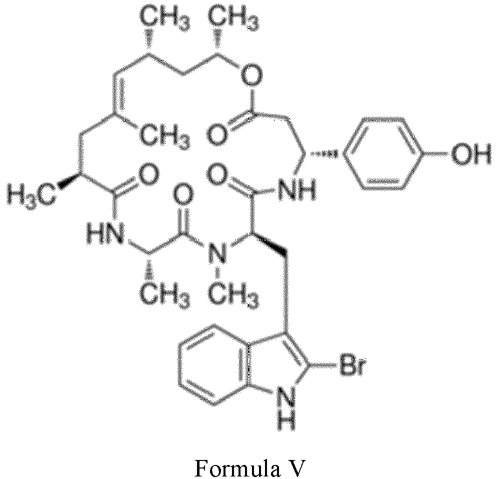
or pharmaceutically acceptable salts thereof; and 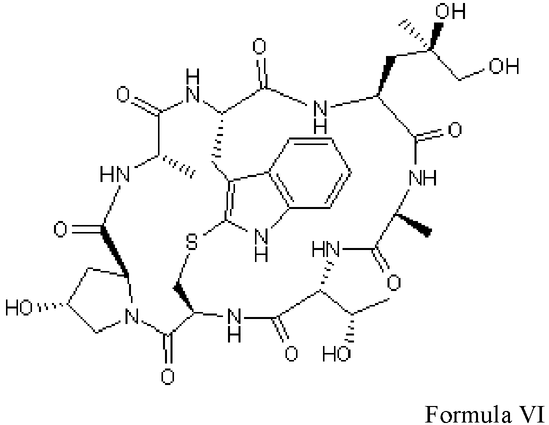 or pharmaceutically acceptable salts thereof.

In some embodiments, the immunomodulatory compound is of Formula I, II, III, IV, V or VI. In some other embodiments, the immunomodulatory compound is of formula I and II. In some other embodiments, the immunomodulatory compound is of formula I, II and III. In some other embodiments, the immunomodulatory compound is of formula I, II, III and IV. In some other embodiments, the immunomodulatory compound is of formula IV, V and VI. In some preferred embodiments, the immunomodulatory compound is of Formula I and III. In further preferred embodiments, the immunomodulatory compound is of Formula I.

In another embodiment of the invention, the immunomodulatory compound is selected from the group comprising 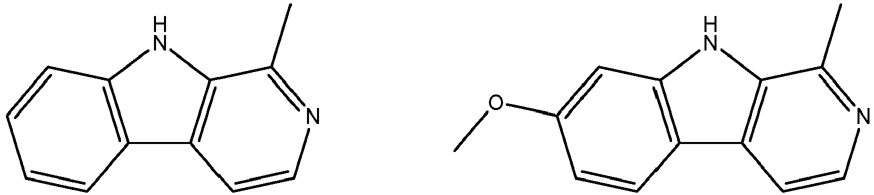 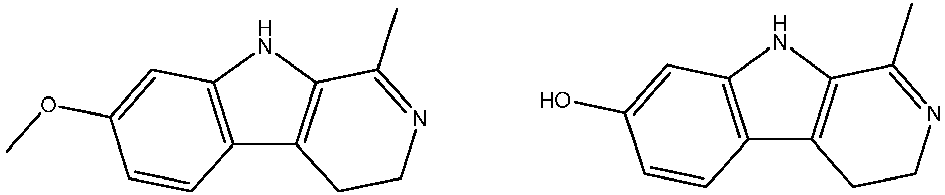 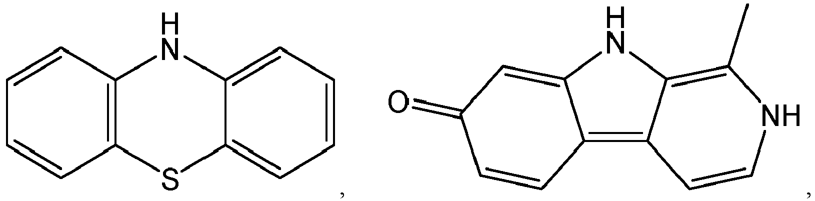 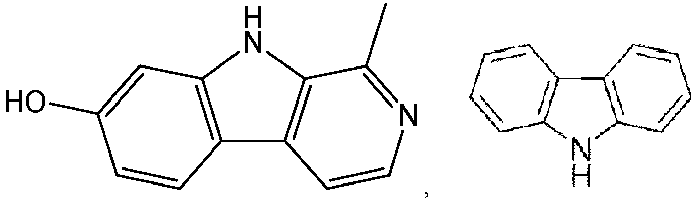 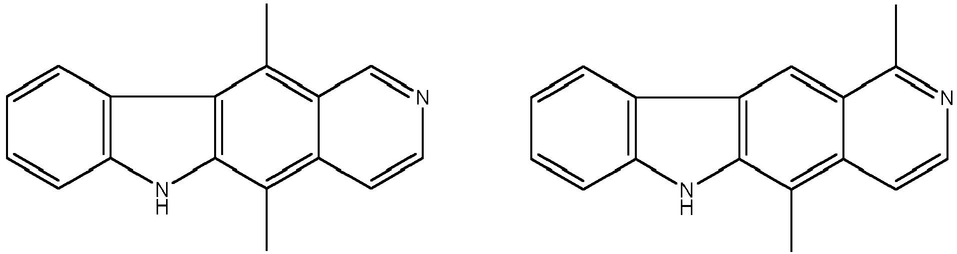 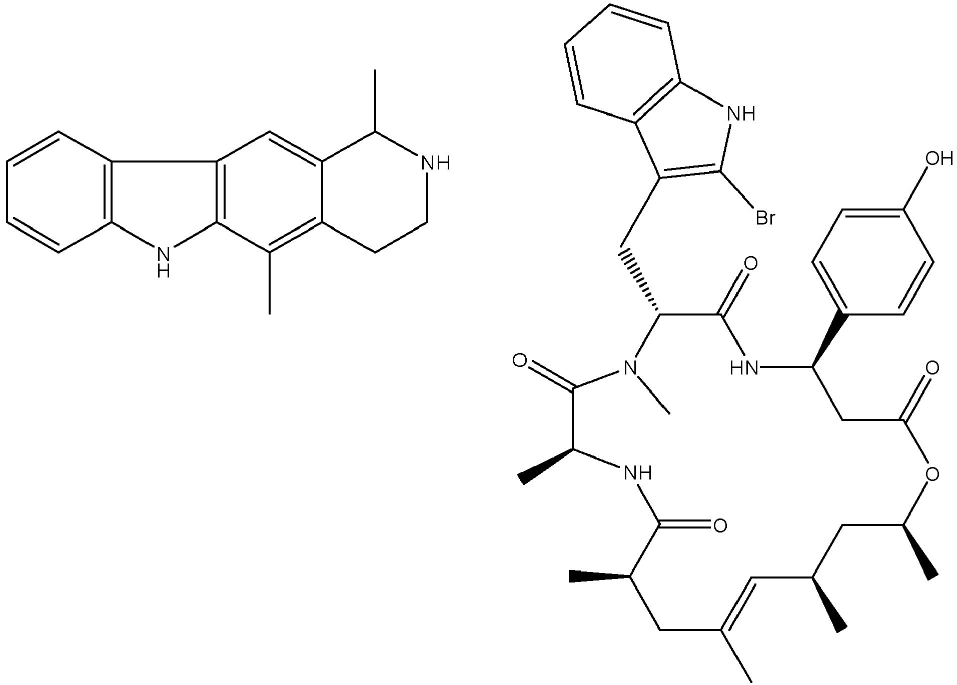 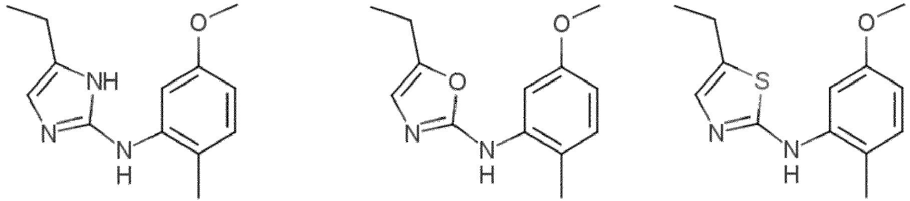 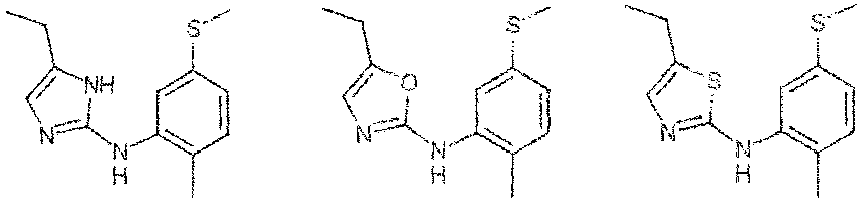 or pharmaceutically acceptable salts thereof.

In another embodiment of the invention, the immunomodulatory compound is selected from the group comprising 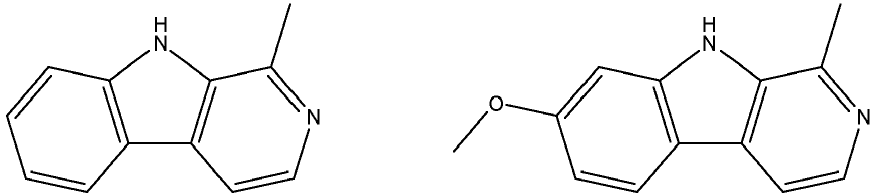 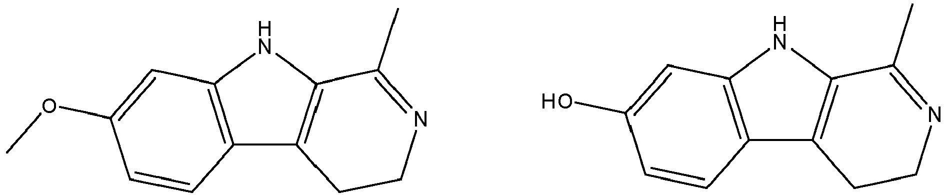 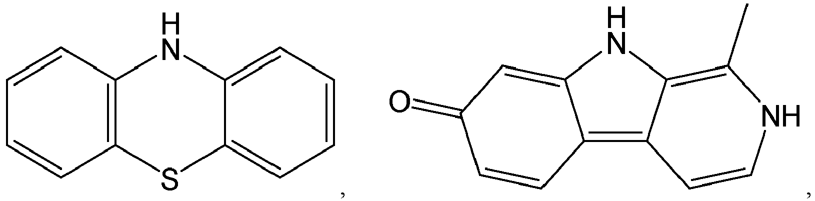 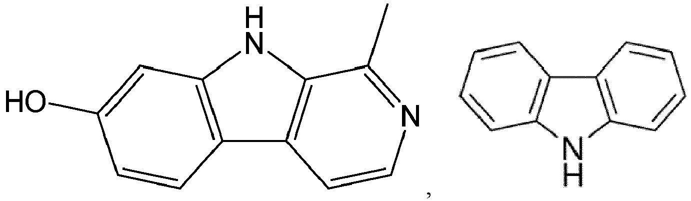 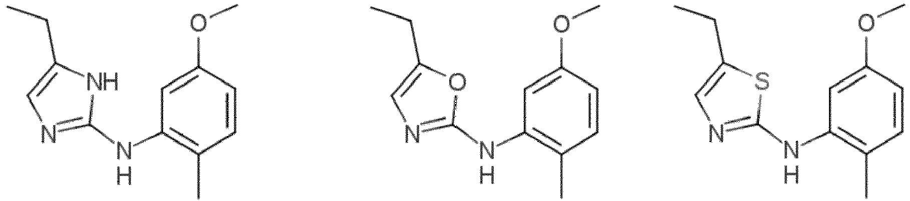 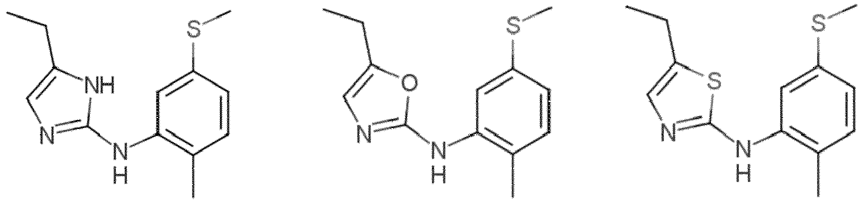 or pharmaceutically acceptable salts thereof.

In a preferred embodiment of the invention, the immunomodulatory compound is selected from the group comprising 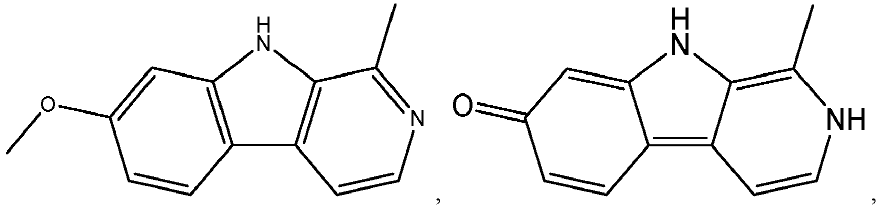 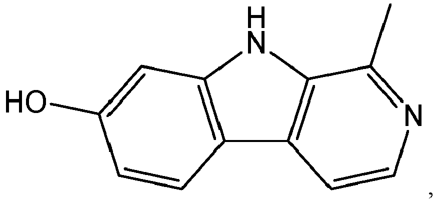 or pharmaceutically acceptable salts thereof

In another embodiment of the invention, the immunomodulatory compound is selected from the group comprising 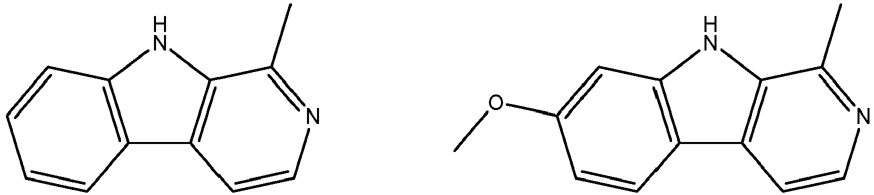 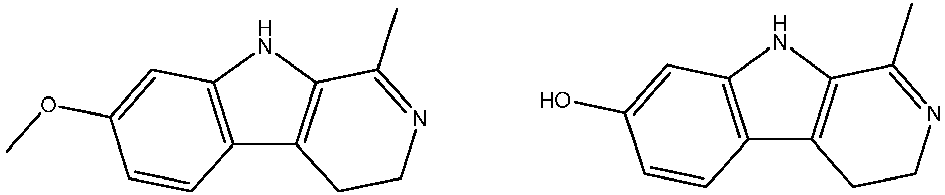 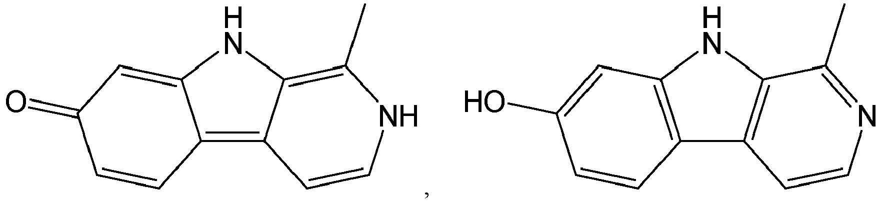 or pharmaceutically acceptable salts thereof.
preferably the immunomodulatory compound is 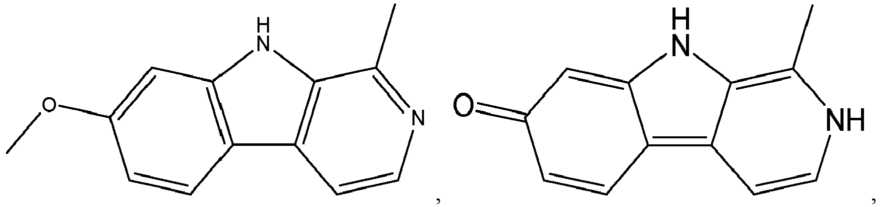 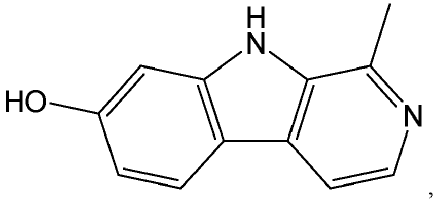 or pharmaceutically acceptable salts thereof.

In another embodiment of the invention, the immunomodulatory compound is selected from the group comprising 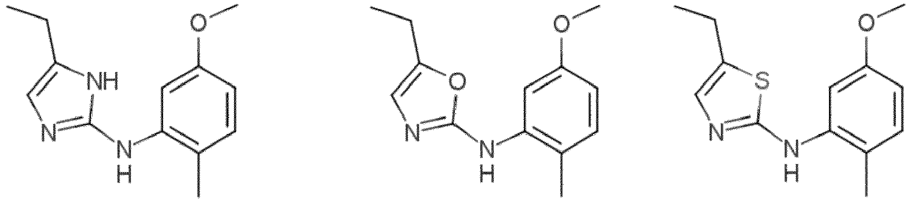 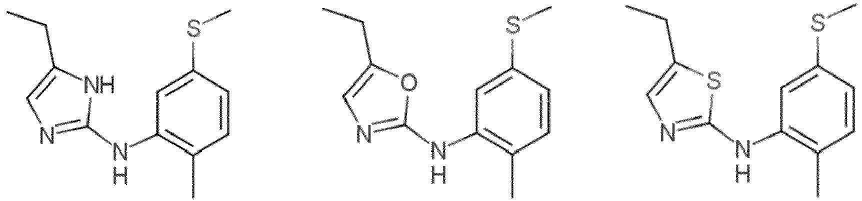 or pharmaceutically acceptable salts thereof.

The present invention also includes pharmaceutically acceptable salts of the immunomodulatory compounds of the invention. As used herein, "pharmaceutically acceptable salts" refers to derivatives of the immunomodulatory compounds wherein the parent compound is modified by converting an existing acid or base moiety to its salt form (e.g., by reacting the free base group with a suitable organic acid). Examples of pharmaceutically acceptable salts include, but are not limited to, mineral or organic acid salts of basic residues such as amines; alkali or organic salts of acidic residues such as carboxylic acids; and the like. Representative acid addition salts include acetate, acetic acid, adipate, alginate, ascorbate, aspartate, benzenesulfonate, benzene sulfonic acid, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate (mesylate), 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, sulfonate, tartrate, thiocyanate, toluenesulfonate, undecanoate, valerate salts, and the like; preferably hydrochloride, mesylate, sulfonate, acetic acid, carboxylic acid and citrate. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. The pharmaceutically acceptable salts of the invention include the conventional non-toxic salts of the parent compound formed, for example, from non-toxic inorganic or organic acids. The pharmaceutically acceptable salts of the invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, Pharmaceutical Salts: Properties, Selection, and Use, P.H. Stahl and C.G. Wermuth (eds.), Wiley-VCH, 2008, and Berge et al, Journal of Pharmaceutical Science, 66, 1-19 (1977), each of which is incorporated herein by reference in its entirety.

The immunomodulatory compounds of the invention are under base form or under water soluble appropriate pharmaceutically acceptable salt, and/or have either amorphous or crystalline form, and/or are under free molecule or linked to an appropriate carrier such as liposome, lipoplexe, or polycyanoacrylate nanoparticles.

The immunomodulatory compounds of the invention can be used as adjuvants or as co-adjuvants, for example in immunogenic compositions or as components in vaccines. Typically, immunogenic compositions disclosed herein include an antigen, at least one immunomodulatory compound of the invention, optionally an additional adjuvant, such as a conventional adjuvant, or a combination thereof. The combination of an adjuvant and an antigen can be referred to as a vaccine. When administered to a subject in combination, the immunomodulatory compounds, a conventional adjuvant and/or antigen can be administered in separate pharmaceutical compositions, or they can be administered together in the same pharmaceutical composition. When administered in combination, the conventional adjuvant can be a lipid conjugate, the antigen can be a lipid conjugate, or the adjuvant and the antigen can both be lipid conjugates.

Limited vaccine efficacy can be ascribed, in part, to transient and/or low expression of antigen in the context of MHC on the cell surface and limited co-stimulatory molecule generation. Cytokine responses are essential for the polarization of the T cell immune response but are unable to effect any potent initiation of adaptive immune responses on their own. The immunomodulatory compounds of the invention modulate immune responses for protective immune response generation through the enhancing formation of the immunological synapse, modulating the expression of immune response associated markers on the cell surface and/or by decreases undesired aspects of the immune response linked with cellular exhaustion and excessive inflammation, such as "cytokine-storm" induced due to high cytokine levels. These aspects are also beneficial for therapeutic applications where immune subversion and/or "cytokine storm play a role in disease pathology, including inflammatory disorders, cancer, auto-immune disease and infection.

The immunomodulatory compounds of the invention have the following properties:
- improve the expression and presentation of antigens in the context of MHC-II and
- MHC-I at the cell surface.
- Affects changes to surface expression markers including increased costimulatory molecule expression
- enhance antigen delivery to the draining lymph node, for priming of T cells, while modulating the cytokine and chemokine response.
- reduces exacerbated cytokine production.
- do not activate TLR

All these properties together improve the ability for dendritic cells, in the context of antigen presentation, not only improve Th2-dependent antibody responses but also that of Th1, Th17 and CD8+ T cell dependent cytotoxic immune responses, respectively. This is of particular interest in developing strategies against infectious intracellular pathogens (e.g.: Mycobacterium tuberculosis, Leishmania spp. and respiratory syncytial virus (RSV)).

Yet, the immunomodulatory compounds of the invention may also serve to improve the immunogenicity of already existing immunogenic compositions, such as vaccine candidates, through enhancing the strength of the resulting B cell responses.

The immunomodulatory compounds can be administered as part of a preventive or therapeutic regimen for:
- Vaccine development against infectious agents, cancer and auto-immunity. They can be either administered with vaccine antigen either alone or in combination with other adjuvents. Examples of such adjuvants can be but are not limited to TLR stimulating compounds (e.g.: PolyI:C, CpG, Monophosphoryl Lipid A- a low-toxic derivative of Lipopolysaccharide (LPS) or Alum)
- An immunotherapy-based strategy to treat clinical disease due to infectious pathogens, cancer and autoimmunity. Here, the administration of the immunomodulatory compounds of the invention function to overcome immune response suppression and combat exacerbated cytokine induced pathology. It can be administered alone or in conjunction with anti-infectives (anti-pathogenic microorganism), or in conjunction with other immunomodulatory agents.

According to the invention, the immunomodulatory compounds of the invention may act alone, for example as an adjuvant or as a co-adjuvant, or in an immunogenic composition of the invention, that can be a vaccine. As used herein, a "vaccine" is a biological preparation that improves immunity to a particular disease, such as cancer or autoimmunity, or improves immunity to an infectious agent. A vaccine introduces an antigen into the tissues or cells of a subject and elicits an immune response, thereby protecting the subject from a particular disease or pathogen infection.

An aspect of the present invention provides an adjuvant composition comprising at least one immunomodulatory compound and at least one pharmaceutically acceptable excipient and/or carrier, wherein the immunomodulatory compound is selected from the group consisting of 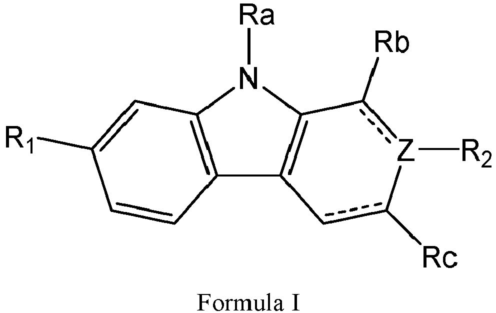 preferably 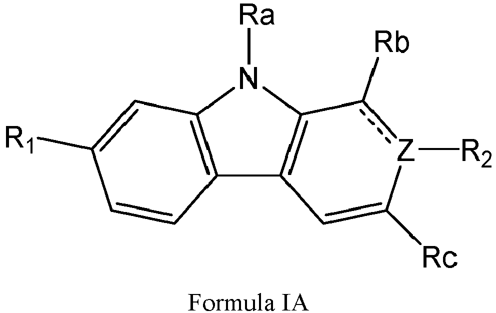 or pharmaceutically acceptable salts thereof
wherein
Z is N or C, preferably Z is N;
R₁ is selected from the group comprising -H, =O, -OH, -S-CH₃, -OCH₃, and -CH₂OH; preferably R₁ is selected from the group comprising -H, =O, -OH, -OCH₃;
R₂ is absent or selected from the group comprising -H, -CH₃, and -CH₂-CH₃; preferably R2 is absent or is -H;
Ra is selected from the group comprising -H, -CH₃ and -(CH₂)n-CH₃, wherein n is 1 to 20; preferably Ra is -H;
Rb is -H or -CH₃;
Rc is -H or -NH₂;
Preferably Z is N and R₂ is absent;
When R₁ is =O, then Z is N and R₂ is -H; 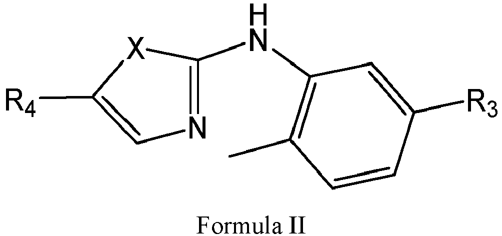 or pharmaceutically acceptable salts thereof
   wherein
   X is selected from the group comprising NH, O, and S;
   R₃ is selected from the group comprising -H, -OH, -OCH₃, -CH₂-OH, and -S-CH₃; preferably R3 is -OCH₃ or -S-CH₃;
   R₄ is selected from the group comprising -CH₃, -CH₂-CH₃, 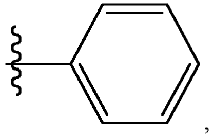 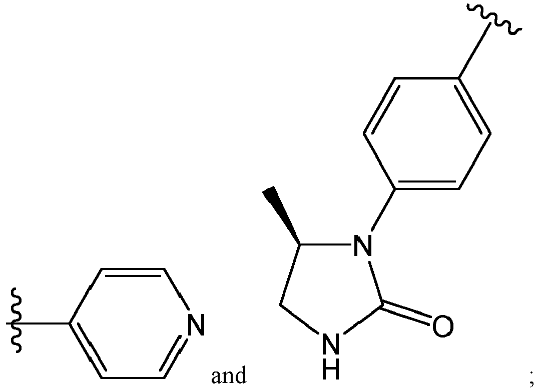 preferably R4 is -CH₃ or -CH₂-CH₃; 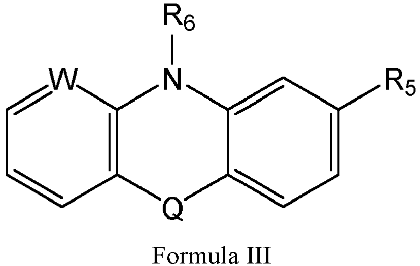 or pharmaceutically acceptable salts thereof
      wherein
      W is N or CH;
      Q is selected from the group comprising O, S and NH;
      R₅ is selected from the group comprising -H, -OH, -OCH₃, -CH₂OH, and -S-CH₃;
      R₆ is selected from the group comprising -H, -CH₃, -CH₂-CH₃, -CH₂-CH₂-N(CH₃)₂, 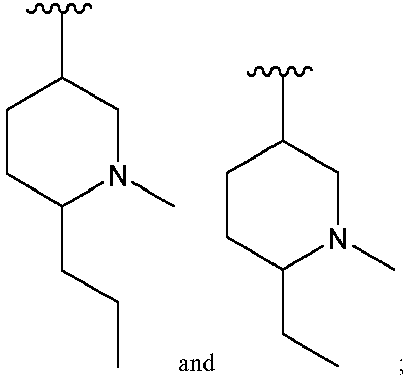 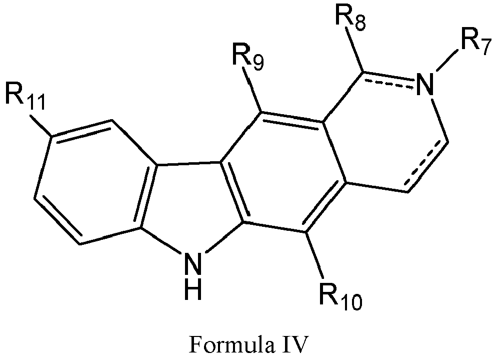 or pharmaceutically acceptable salts thereof
         wherein
         R7 is absent or selected from the group comprising -H, -CH₃ and -CH₂-CH₃;
         R8 is -H or -CH₃;
         R9 is -H or -CH₃;
         R10 is -H or CH₃;
         R11 is selected from the group comprising -H, -OH and -O-CH₃; 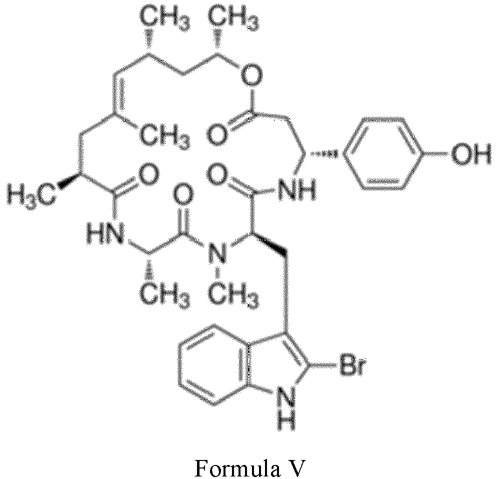
or pharmaceutically acceptable salts thereof; and 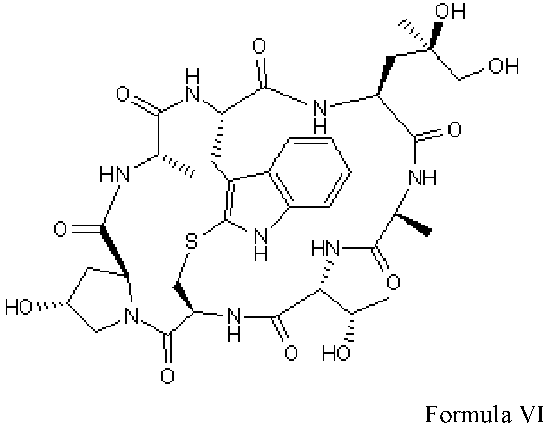 or pharmaceutically acceptable salts thereof.

In some embodiments, the immunomodulatory compound in the adjuvant composition of the invention is of Formula I, II, III, IV, V or VI. In some other embodiments, the immunomodulatory compound in the immunogenic composition of the invention is of formula I and II. In some other embodiments, the immunomodulatory compound in the immunogenic composition of the invention is of formula I, II and III. In some other embodiments, the immunomodulatory compound in the immunogenic composition of the invention is of formula I, II, III and IV. In some other embodiments, the immunomodulatory compound in the immunogenic composition of the invention is of formula IV, V and VI. In some preferred embodiments, the immunomodulatory compound in the immunogenic composition of the invention is of Formula I and III. In further preferred embodiments, the immunomodulatory compound in the immunogenic composition of the invention is of Formula I.

The adjuvant composition of the invention can further comprise one or more conventional adjuvants (or immune potentiators). Conventional adjuvants useful in the present invention may include, but are not limited to, natural or synthetic. They may be organic or inorganic. Adjuvants may be selected from any of the classes (1) mineral salts, e.g., aluminium hydroxide and aluminium or calcium phosphate gels; (2) emulsions including: oil emulsions and surfactant based formulations, e.g., microfluidised detergent stabilised oil-in-water emulsion, purified saponin, oil-in-water emulsion, stabilised water-in-oil emulsion; (3) particulate adjuvants, e.g., virosomes (unilamellar liposomal vehicles incorporating influenza haemagglutinin), structured complex of saponins and lipids, polylactide co-glycolide (PLG); (4) microbial derivatives; (5) endogenous human immunomodulators; and/or (6) inert vehicles, such as gold particles; (7) microorganism derived adjuvants; (8) tensoactive compunds; (9) carbohydrates; or combinations thereof.

In some embodiments, the adjuvant composition of the invention further comprises one or more conventional adjuvants selected from the group comprising cationic liposome-DNA complex JVRS-100, aluminum hydroxide vaccine adjuvant, aluminum phosphate vaccine adjuvant, aluminum potassium sulfate adjuvant, alhydrogel, ISCOM(s)^{™}, Freund's Complete Adjuvant, Freund's Incomplete Adjuvant, CpG DNA Vaccine Adjuvant, Poly I:C, Cholera toxin, Cholera toxin B subunit, Liposomes, Saponin Vaccine Adjuvant, DDA Adjuvant, Squalene-based Adjuvants, Etx B subunit Adjuvant, IL-12 Vaccine Adjuvant, LTK63 Vaccine Mutant Adjuvant, TiterMax Gold Adjuvant, Ribi Vaccine Adjuvant, Montanide ISA 720 Adjuvant, Corynebacterium-denved P40 Vaccine Adjuvant, MPL^{™} Adjuvant, AS04, AS02, Lipopolysaccharide Vaccine Adjuvant, Muramyl Dipeptide Adjuvant, CRL1005, Killed Corymb acterium parvum Vaccine Adjuvant, Montanide ISA 51, Bordetella pertussis component Vaccine Adjuvant, Cationic Liposomal Vaccine Adjuvant, Adamantylamide Dipeptide Vaccine Adjuvant, Arlacel A, VSA-3 Adjuvant, Aluminum vaccine adjuvant, Polygen Vaccine Adjuvant, ADJUMER^{™}, Algal Glucan, Bay R1005, Theramide^{®}, Stearyl Tyrosine, Specol, Algammulin, AVRIDINE^{®}, Calcium Phosphate Gel, CTA1-DD gene fusion protein, DOC/Alum Complex, Gamma Inulin, Gerbu Adjuvant, GM-CSF, GMDP, Recombinant hlFN-gamma/Interferon-g, Interleukin- 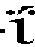 β, Inter leukin-2, Interleukin-7, Sclavo peptide, Rehydragel LV, Rehydragel HP A, Loxoribine, MF59, MTP-PE Liposomes, Murametide, Murapalmitine, D-Murapalmitine, NAGO, Non-Ionic Surfactant Vesicles, PMMA, Protein Cochleates, QS-21, SPT (Antigen Formulation), nanoemulsion vaccine adjuvant, AS03, Quil-A vaccine adjuvant, RC529 vaccine adjuvant, LTR192G Vaccine Adjuvant, E. coli heat-labile toxin, LT, amorphous aluminum hydroxyphosphate sulfate adjuvant, Calcium phosphate vaccine adjuvant, Montanide Incomplete Seppic Adjuvant, Imiquimod, Resiquimod, AF03, Flagellin, Poly(LC), ISCOMATRIX^{®}, Abisco-100 vaccine adjuvant, Albumin-heparin microparticles vaccine adjuvant, AS-2 vaccine adjuvant, B7-2 vaccine adjuvant, DHEA vaccine adjuvant, Immunoliposomes Containing Antibodies to Costimulatory Molecules, SAF-1, Sendai Proteo liposomes, Sendai-containing Lipid Matrices, Threonyl muramyl dipeptide (TMDP), Ty Particles vaccine adjuvant, Bupivacaine vaccine adjuvant, DL-PGL (Polyester poly (DL-lactide-co-glycolide)) vaccine adjuvant, IL-15 vaccine adjuvant, LTK72 vaccine adjuvant, MPL-SE vaccine adjuvant, non-toxic mutant E112K of Cholera Toxin mCT-El 12K, and/or Monophosphoryl lipid A (MPLA), Matrix-S.

In other embodiments, the adjuvant composition of the invention further comprises one or more conventional adjuvants selected from the group comprising cationic liposome-DNA complex JVRS-100, aluminum hydroxide vaccine adjuvant, aluminum phosphate, aluminum potassium sulfate, alhydrogel, Freund's Complete Adjuvant, Freund's Incomplete Adjuvant, CpG DNA, Poly I:C, cholera toxin, cholera toxin B subunit, saponin, DDA, Squalene-based adjuvants, Etx B subunit adjuvant, IL-12 vaccine adjuvant, LTK63 vaccine mutant adjuvant, Ribi vaccine adjuvant, Corynebacterium-derived P40 vaccine adjuvant, lipopolysaccharide vaccine adjuvant, muramyl dipeptide adjuvant, killed Corynebacterium parvum vaccine adjuvant, Bordetella pertussis component vaccine adjuvant, cationic Liposomal vaccine adjuvant, Adamantylamide Dipeptide vaccine adjuvant, Aluminum vaccine adjuvant, Algal Glucan, Stearyl Tyrosine, Specol, Algammulin, Calcium Phosphate Gel, CTA1-DD gene fusion protein, DOC/ Alum Complex, Gamma Inulin, Gerbu Adjuvant, GM-CSF, GMDP, Recombinant hlFN-gamma/Interferon-g, Interleukin- 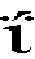 β, Interleukin-2, Interleukin-7, Sclavo peptide, Rehydragel LV, Rehydragel HPA, Loxoribine, MF59, MTP-PE Liposomes, Murametide, Murapalmitine, D-Murapalmitine, NAGO, Non-Ionic Surfactant Vesicles, PMMA, Protein Cochleates, QS-21, SPT (Antigen Formulation), nanoemulsion vaccine adjuvant, Quil-A vaccine adjuvant, RC529 vaccine adjuvant, LTR192G vaccine adjuvant, E. coli heat-labile toxin, LT, amorphous aluminum hydroxyphosphate sulfate adjuvant, Calcium phosphate vaccine adjuvant, Imiquimod, Resiquimod, Flagellin, Poly(LC), Albumin-heparin microparticles vaccine adjuvant, AS-2 vaccine adjuvant, B7-2 vaccine adjuvant, DHEA vaccine adjuvant, Immunoliposomes Containing Antibodies to Costimulatory Molecules, SAF-1, Sendai Proteo liposomes, Sendai-containing Lipid Matrices, Threonyl muramyl dipeptide (TMDP), Ty Particles vaccine adjuvant, Bupivacaine vaccine adjuvant, DL-PGL (Polyester poly (DL-lactide-co-glycolide)) vaccine adjuvant, IL-15 vaccine adjuvant, LTK72 vaccine adjuvant, MPL-SE vaccine adjuvant, non-toxic mutant El 12K of Cholera Toxin mCT-El 12K, Monophosphoryl lipid A (MPLA) and/or Matrix-S.

Another aspect of the present invention provides an immunogenic composition comprising an antigen and one or more immunomodulatory compounds, wherein the immunomodulatory compound is selected from the group consisting of 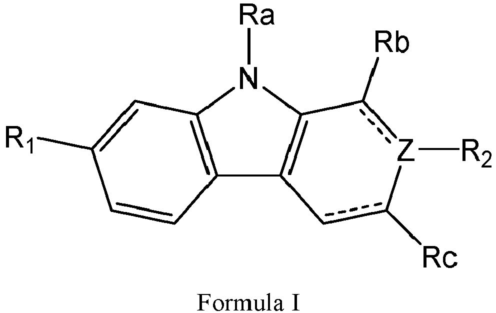 preferably 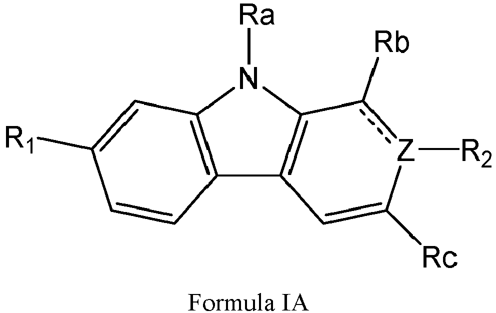 or pharmaceutically acceptable salts thereof
wherein
Z is N or C; preferably Z is N;
R₁ is selected from the group comprising -H, =O, -OH, -S-CH₃, -OCH₃, and -CH₂OH; preferably R₁ is selected from the group comprising -H, =O, -OH, -OCH₃;
R₂ is absent or selected from the group comprising -H, -CH₃, and -CH₂-CH₃; preferably R₂ is absent or is -H;
Ra is selected from the group comprising -H, -CH₃ and -(CH₂)n-CH₃, wherein n is 1 to 20 or 1 to 10, preferably n is 1 to 5 or 1 to 3; preferably Ra is -H
Rb is -H or -CH₃;
Rc is -H or -NH₂;
Preferably Z is N and R₂ is absent;
When R₁ is =O, then Z is N and R₂ is -H; 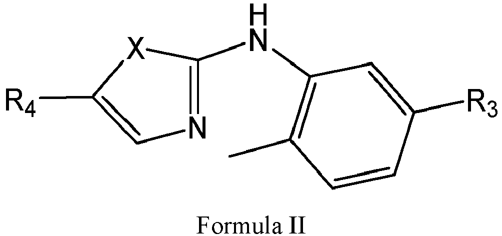 or pharmaceutically acceptable salts thereof
   wherein
   X is selected from the group comprising NH, O, and S;
   R₃ is selected from the group comprising -H, -OH, -OCH₃, -CH₂-OH, and -S-CH₃; preferably R3 is -OCH₃ or -S-CH₃;
   R₄ is selected from the group comprising -CH₃, -CH₂-CH₃, 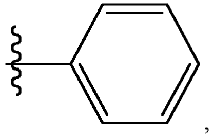 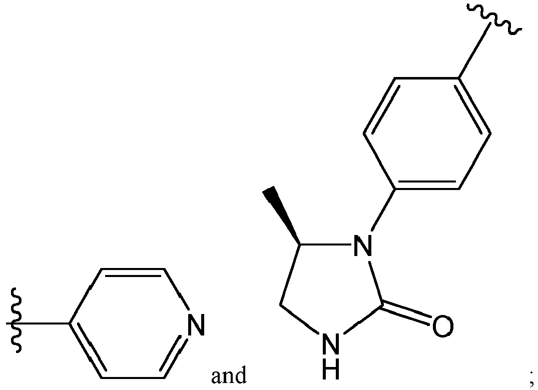 preferably R4 is -CH₃ or -CH₂-CH₃; 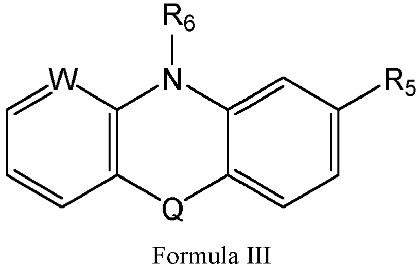 or pharmaceutically acceptable salts thereof
      wherein
      W is N or CH; preferably W is CH;
      Q is selected from the group comprising O, S and NH; preferably Q is S;
      R₅ is selected from the group comprising -H, -OH, -OCH₃, -CH₂OH, and -S-CH₃; preferably R₅ is -H;
      R₆ is selected from the group comprising -H, -CH₃, -CH₂-CH₃, -CH₂-CH₂-N(CH₃)₂, 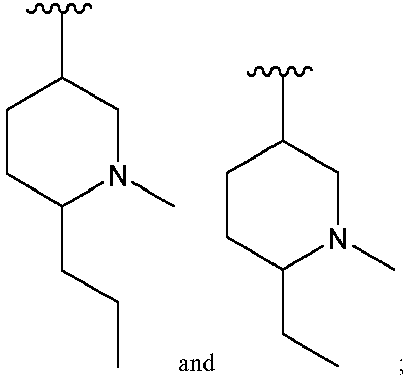 preferably R₆ is -H. 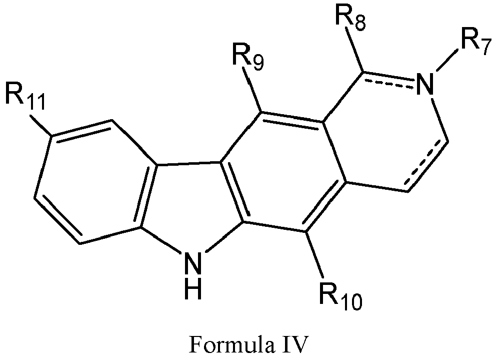 or pharmaceutically acceptable salts thereof
         wherein
         R7 is absent or selected from the group comprising -H, -CH₃ and -CH₂-CH₃;
         R8 is -H or -CH₃;
         R9 is -H or -CH₃; preferably R9 is -CH₃;
         R10 is -H or CH₃; preferably R10 is -CH₃;
         R11 is selected from the group comprising -H, -OH and -O-CH₃; 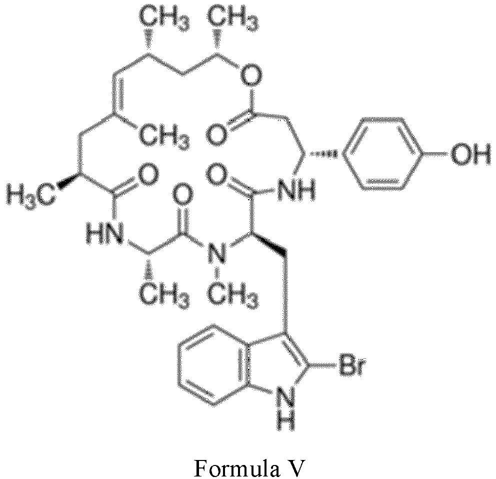
or pharmaceutically acceptable salts thereof; and 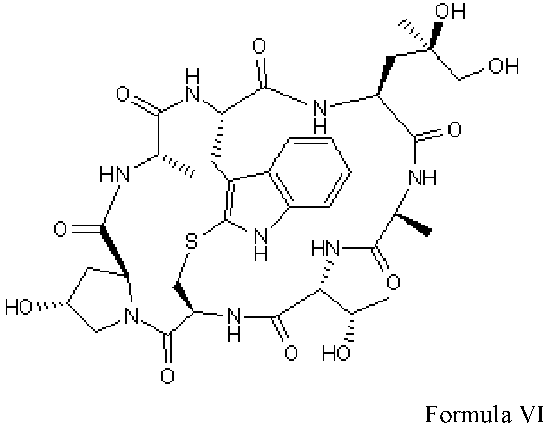 or pharmaceutically acceptable salts thereof.

In some embodiments, the immunomodulatory compound in the immunogenic composition of the invention is of Formula I, II, III, IV, V or VI. In some other embodiments, the immunomodulatory compound in the immunogenic composition of the invention is of formula I and II. In some other embodiments, the immunomodulatory compound in the immunogenic composition of the invention is of formula I, II and III. In some other embodiments, the immunomodulatory compound in the immunogenic composition of the invention is of formula I, II, III and IV. In some other embodiments, the immunomodulatory compound in the immunogenic composition of the invention is of formula IV, V and VI. In some preferred embodiments, the immunomodulatory compound in the immunogenic composition of the invention is of Formula I and III. In further preferred embodiments, the immunomodulatory compound in the immunogenic composition of the invention is of Formula I.

In some embodiments of the immunogenic composition of the invention, the immunomodulatory compound is selected from the group comprising 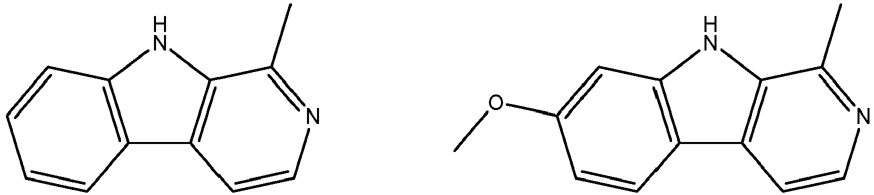 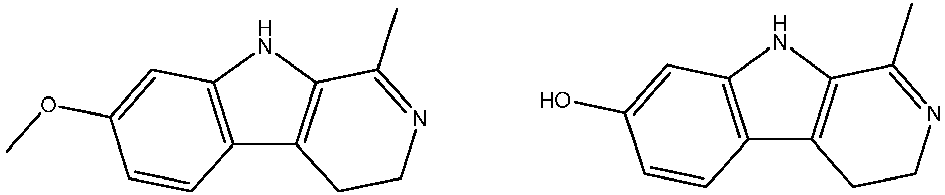 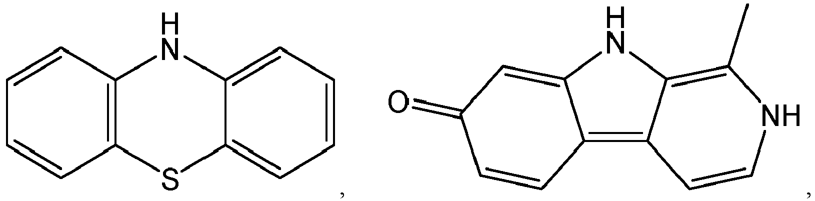 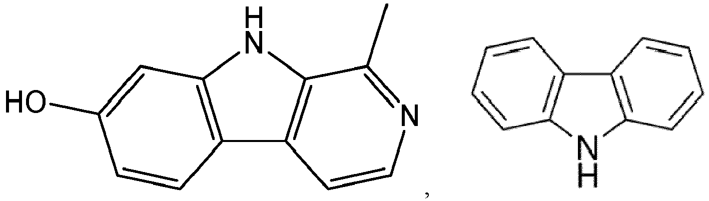 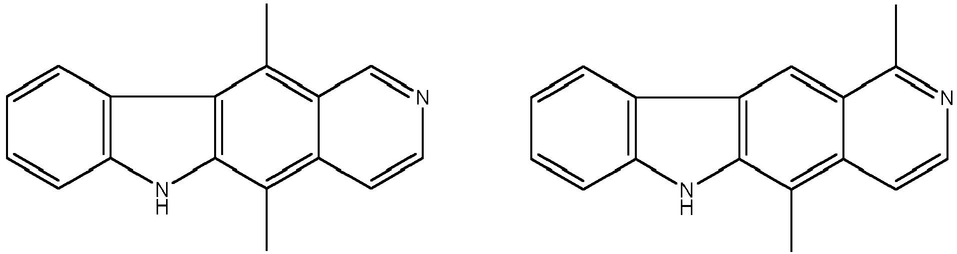 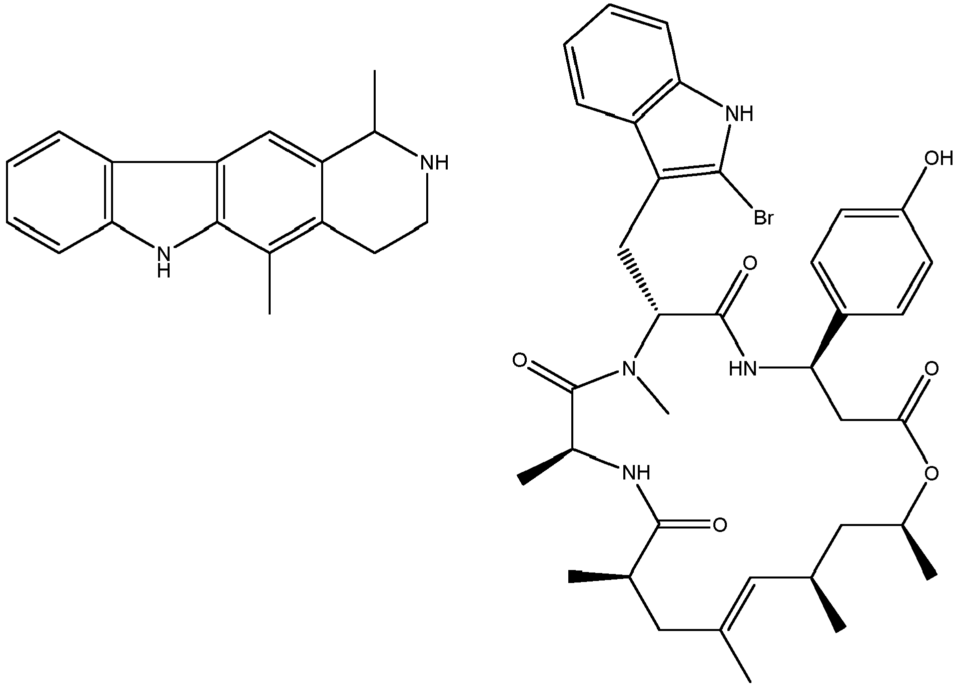 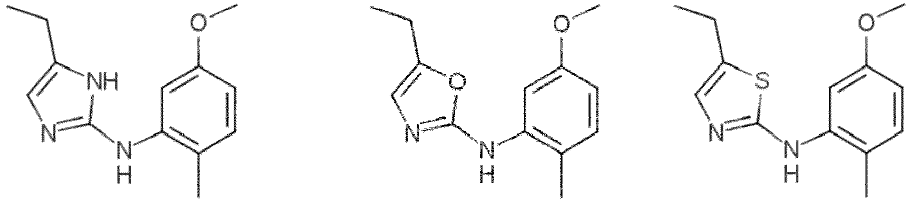 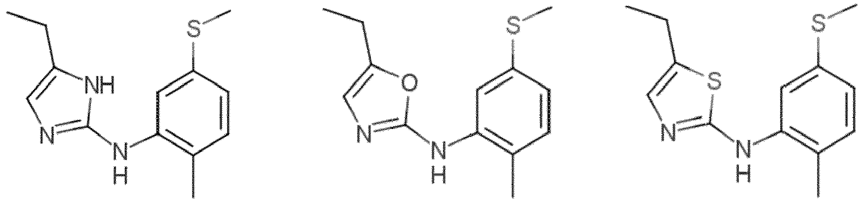 or pharmaceutically acceptable salts thereof.

In some other embodiments of the immunogenic composition of the invention, the immunomodulatory compound is selected from the group comprising 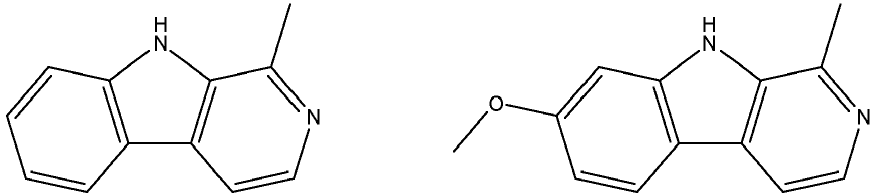 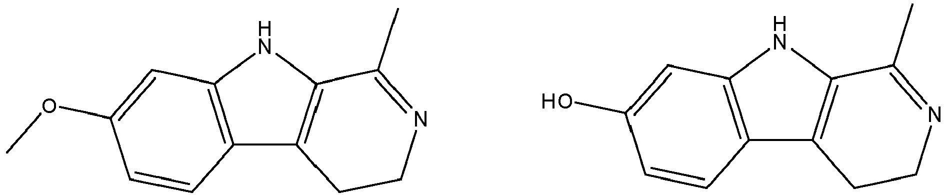 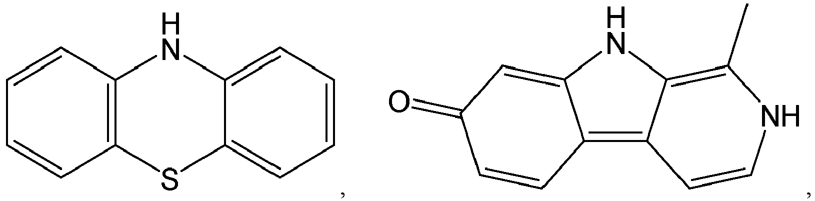 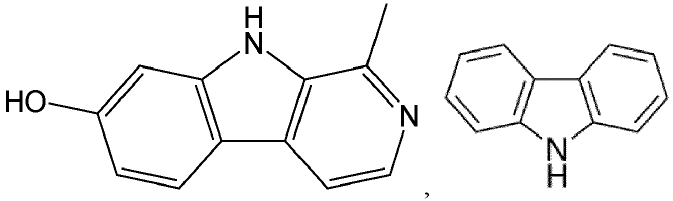 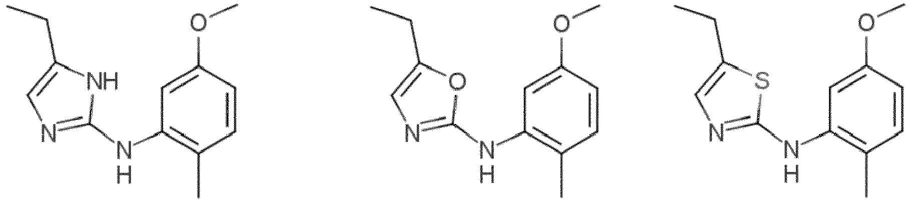 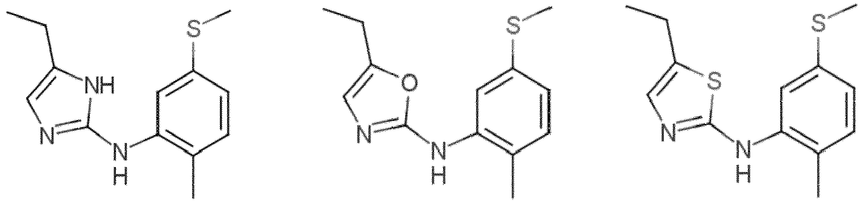 or pharmaceutically acceptable salts thereof.

In a preferred embodiment of the immunogenic composition the invention, the immunomodulatory compound is selected from the group comprising 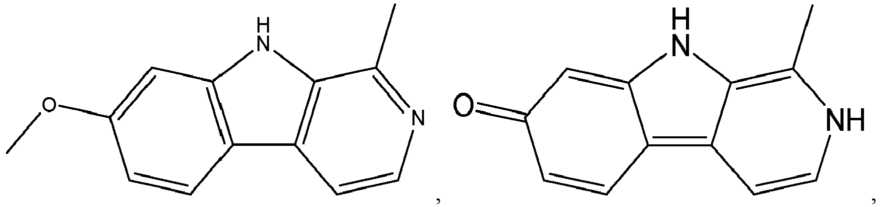 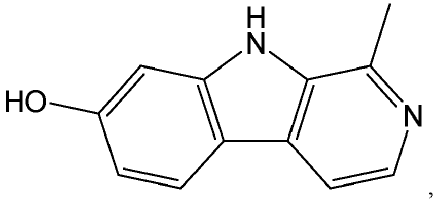 or pharmaceutically acceptable salts thereof.

In another embodiment of the immunogenic composition of the invention, the immunomodulatory compound is selected from the group comprising 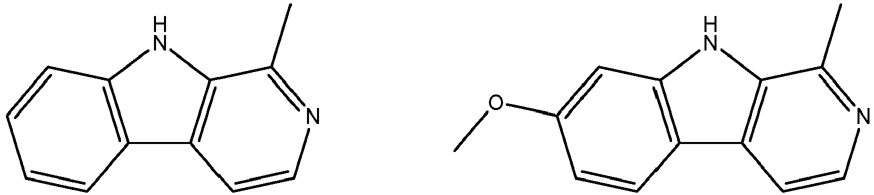 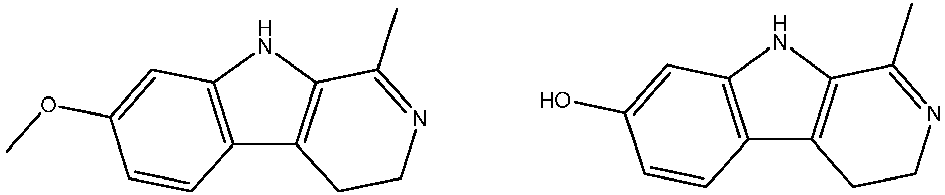 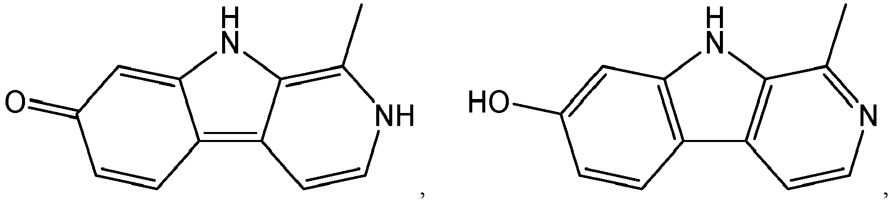 or pharmaceutically acceptable salts thereof
preferably the immunomodulatory compound is 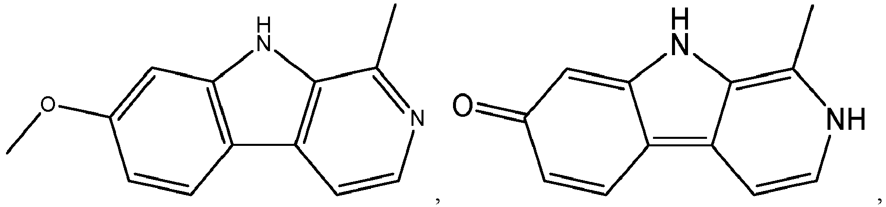 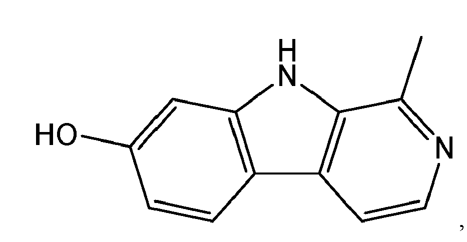 or pharmaceutically acceptable salts thereof.

In another preferred embodiment of the immunogenic composition of the invention, the immunomodulatory compound is selected from the group comprising 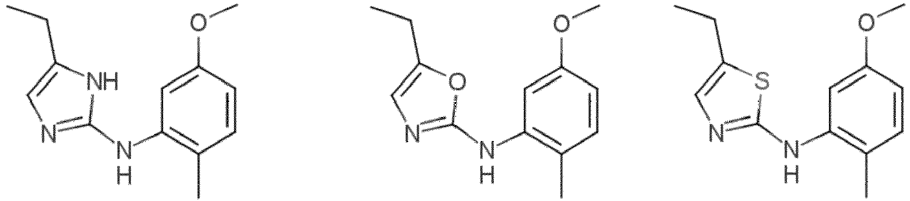 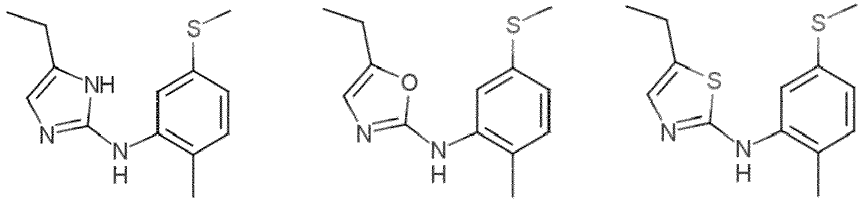 or pharmaceutically acceptable salts thereof.

Antigens of the immunogenic composition of the invention can be peptides, proteins, polysaccharides, saccharides, lipids, nucleic acids, or combinations thereof. The antigen can be derived from a virus, bacterium, parasite, plant, protozoan, fungus, tissue or transformed cell such as a cancer or leukemic cell and/or can be a whole cell, a whole inactivated or attenuated organisms or immunogenic component thereof, e.g., cell wall components or molecular components thereof. These organisms may be infectious organisms, such as viruses, parasites and bacteria. These organisms may also be tumor cells. The antigens may be purified or partially purified polypeptides derived from tumors or viral or bacterial sources. The antigens can be recombinant polypeptides produced by expressing DNA encoding the polypeptide antigen in a heterologous expression system. The antigens can be DNA encoding all or part of an antigenic protein. The DNA may be in the form of vector DNA such as plasmid DNA. Antigens may be provided as single antigens or may be provided in combination. Antigens may also be provided as complex mixtures of polypeptides or nucleic acids. Suitable antigens are known in the art and are available from market and scientific sources. Exemplary antigens are provided below:
Viral antigens : a viral antigen can be isolated from any virus including, but not limited to, a virus from any of the following viral families: Arenaviridae, Arterivirus, Astroviridae, Baculoviridae, Badnavirus, Barnaviridae, Birnaviridae, Bromoviridae, Bunyaviridae, Caliciviridae, Capillovirus, Carlavirus, Caulimovirus, Circoviridae, Closterovirus, Comoviridae, Coronaviridae (e.g., Coronavirus, such as severe acute respiratory syndrome (SARS) virus), Corticoviridae, Cystoviridae, Deltavirus, Dianthovirus, Enamovirus, Filoviridae (e.g., Marburg virus and Ebola virus (e.g., Zaire, Reston, Ivory Coast, or Sudan strain)), Flaviviridae, (e.g., Hepatitis C virus, Dengue virus 1, Dengue virus 2, Dengue virus 3, and Dengue virus 4), Hepadnaviridae, Herpesviridae (e.g., Human herpesvirus 1, 3, 4, 5, and 6, and Cytomegalovirus), Hypoviridae, Iridoviridae, Leviviridae, Lipothrixviridae, Microviridae, Orthomyxoviridae (e.g., Influenzavirus A and B and C), Papovaviridae, Paramyxoviridae (e.g., measles, mumps, and human respiratory syncytial virus), Parvoviridae, Picornaviridae (e.g., poliovirus, rhinovirus, hepatovirus, and aphthovirus), Poxviridae (e.g. , vaccinia and smallpox virus), Reoviridae (e.g., rotavirus), Retroviridae (e.g., lentivirus, such as human immunodeficiency virus (HIV) 1 and HIV 2), Rhabdoviridae (for example, rabies virus, measles virus, respiratory syncytial virus, etc.), Togaviridae (for example, rubella virus, dengue virus, etc.), and Totiviridae. Suitable viral antigens also include all or part of Dengue protein M, Dengue protein E, Dengue D1NS1, Dengue D1NS2, and Dengue D1NS3. Viral antigens may be derived from a particular strain such as a papilloma virus, a herpes virus, e.g., herpes simplex 1 and 2; a hepatitis virus, for example, hepatitis A virus (HAV), hepatitis B virus (HBV), hepatitis C virus (HCV), the delta hepatitis D virus (HDV), hepatitis E virus (HEV) and hepatitis G virus (HGV), the tick-borne encephalitis viruses; parainfluenza, varicella-zoster, cytomeglavirus, Epstein-Barr, rotavirus, rhinovirus, adenovirus, coxsackieviruses, equine encephalitis, Japanese encephalitis, yellow fever, Rift Valley fever,and lymphocytic choriomeningitis.

Anti-Microbial Polypeptides: Anti-microbial polypeptides (AMPs) are small peptides of variable length, sequence and structure with broad spectrum activity against a wide range of microorganisms including, but not limited to, bacteria, viruses, fungi, protozoa, parasites, prions, and tumor/cancer cells. It has been shown that AMPs have broad-spectrum of rapid onset of killing activities, with potentially low levels of induced resistance and concomitant broad anti-inflammatory effects. In some embodiments, the anti-microbial polypeptide (e.g., an anti-bacterial polypeptide) may be under 10kDa, e.g., under 8kDa, 6kDa, 4kDa, 2kDa, or 1kDa. In some embodiments, the anti-microbial polypeptide (e.g., an anti-bacterial polypeptide) consists of from about 6 to about 100 amino acids, e.g., from about 6 to about 75 amino acids, about 6 to about 50 amino acids, about 6 to about 25 amino acids, about 25 to about 100 amino acids, about 50 to about 100 amino acids, or about 75 to about 100 amino acids. In certain embodiments, the anti-microbial polypeptide (e.g., an anti-bacterial polypeptide) may consist of from about 15 to about 45 amino acids. In some embodiments, the anti-microbial polypeptide (e.g., an anti-bacterial polypeptide) is substantially cationic. In some embodiments, the antimicrobial polypeptide (e.g., an anti-bacterial polypeptide) may be substantially amphipathic. In certain embodiments, the anti-microbial polypeptide (e.g., an anti-bacterial polypeptide) may be substantially cationic and amphipathic. In some embodiments, the anti-microbial polypeptide (e.g., an anti-bacterial polypeptide) may be cytostatic to a Gram-positive bacterium. In some embodiments, the anti-microbial polypeptide (e.g., an anti-bacterial polypeptide) may be cytotoxic to a Gram-positive bacterium. In some embodiments, the antimicrobial polypeptide (e.g., an anti-bacterial polypeptide) may be cytostatic and cytotoxic to a Gram-positive bacterium. In some embodiments, the antimicrobial polypeptide (e.g., an antibacterial polypeptide) may be cytostatic to a Gram-negative bacterium. In some embodiments, the anti-microbial polypeptide (e.g., an anti-bacterial polypeptide) may be cytotoxic to a Gram-negative bacterium. In some embodiments, the antimicrobial polypeptide (e.g., an anti-bacterial polypeptide) may be cytostatic and cytotoxic to a Gram-positive bacterium. In some embodiments, the anti-microbial polypeptide may be cytostatic to a virus, fungus, protozoan, parasite, prion, or a combination thereof. In some embodiments, the anti-microbial polypeptide may be cytotoxic to a virus, fungus, protozoan, parasite, prion, or a combination thereof. In certain embodiments, the anti-microbial polypeptide may be cytostatic and cytotoxic to a virus, fungus, protozoan, parasite, prion, or a combination thereof. In some embodiments, the antimicrobial polypeptide may be cytotoxic to a tumor or cancer cell (e.g., a human tumor and/or cancer cell). In some embodiments, the antimicrobial polypeptide may be cytostatic to a tumor or cancer cell (e.g., a human tumor and/or cancer cell). In certain embodiments, the antimicrobial polypeptide may be cytotoxic and cytostatic to a tumor or cancer cell (e.g., a human tumor or cancer cell). In some embodiments, the anti-microbial polypeptide (e.g., an antibacterial polypeptide) may be a secreted polypeptide. In some embodiments, the anti-microbial polypeptide comprises or consists of a defensin. Exemplary defensins include, but are not limited to, α-defensins (e.g., neutrophil defensin 1, defensin alpha 1, neutrophil defensin 3, neutrophil defensin 4, defensin 5, defensin 6), β-defensins (e.g., beta-defensin 1, beta-defensin 2, beta-defensin 103, beta-defensin 107, beta-defensin 110, beta-defensin 136), and θ-defensins. In other embodiments, the antimicrobial polypeptide comprises or consists of a cathelicidin (e.g., hCAP18).

Anti-Viral Polypeptides: anti-viral polypeptides (AVPs) are small peptides of variable length, sequence and structure with broad spectrum activity against a wide range of viruses. It has been shown that AVPs have a broad-spectrum of rapid onset of killing activities, with potentially low levels of induced resistance and concomitant broad anti- inflammatory effects. In some embodiments, the anti-viral polypeptide is under 10kDa, e.g., under 8kDa, 6kDa, 4kDa, 2kDa, or 1kDa. In some embodiments, the anti-viral polypeptide comprises or consists of from about 6 to about 100 amino acids, e.g., from about 6 to about 75 amino acids, about 6 to about 50 amino acids, about 6 to about 25 amino acids, about 25 to about 100 amino acids, about 50 to about 100 amino acids, or about 75 to about 100 amino acids. In certain embodiments, the anti-viral polypeptide comprises or consists of from about 15 to about 45 amino acids. In some embodiments, the anti-viral polypeptide is substantially cationic. In some embodiments, the anti-viral polypeptide is substantially amphipathic. In certain embodiments, the anti-viral polypeptide is substantially cationic and amphipathic. In some embodiments, the anti-viral polypeptide is cytostatic to a virus. In some embodiments, the anti-viral polypeptide is cytotoxic to a virus. In some embodiments, the anti-viral polypeptide is cytostatic and cytotoxic to a virus. In some embodiments, the anti-viral polypeptide is cytostatic to a bacterium, fungus, protozoan, parasite, prion, or a combination thereof. In some embodiments, the anti-viral polypeptide is cytotoxic to a bacterium, fungus, protozoan, parasite, prion or a combination thereof. In certain embodiments, the anti-viral polypeptide is cytostatic and cytotoxic to a bacterium, fungus, protozoan, parasite, prion, or a combination thereof. In some embodiments, the anti-viral polypeptide is cytotoxic to a tumor or cancer cell (e.g., a human cancer cell). In some embodiments, the anti-viral polypeptide is cytostatic to a tumor or cancer cell (e.g., a human cancer cell). In certain embodiments, the anti-viral polypeptide is cytotoxic and cytostatic to a tumor or cancer cell (e.g., a human cancer cell). In some embodiments, the anti-viral polypeptide is a secreted polypeptide.

Conventional adjuvants (or immune potentiators) may also be administered with or in combination with the immunogenic composition of the invention. Advantages of adjuvants include the enhancement of the immunogenicity of antigens, modification of the nature of the immune response, the reduction of the antigen amount needed for a successful immunization, the reduction of the frequency of booster immunizations needed and an improved immune response in elderly and immunocompromised vaccinees. These may be co-administered by any route, e.g., intramuscularly, subcutaneous, IV or intradermal injections.

Adjuvants useful in the present invention may include, but are not limited to, natural or synthetic. They may be organic or inorganic. Adjuvants may be selected from any of the classes (1) mineral salts, e.g., aluminium hydroxide and aluminium or calcium phosphate gels; (2) emulsions including: oil emulsions and surfactant based formulations, e.g., microfluidised detergent stabilised oil-in-water emulsion, purified saponin, oil-in-water emulsion, stabilised water-in-oil emulsion; (3) particulate adjuvants, e.g., virosomes (unilamellar liposomal vehicles incorporating influenza haemagglutinin), structured complex of saponins and lipids, polylactide co-glycolide (PLG); (4) microbial derivatives; (5) endogenous human immunomodulators; and/or (6) inert vehicles, such as gold particles; (7) microorganism derived adjuvants; (8) tensoactive compunds; (9) carbohydrates; or combinations thereof.

In some embodiments, the immunogenic composition of the invention further comprises one or more adjuvants selected from the group comprising cationic liposome-DNA complex JVRS-100, aluminum hydroxide vaccine adjuvant, aluminum phosphate vaccine adjuvant, aluminum potassium sulfate adjuvant, alhydrogel, ISCOM(s)^{™}, Freund's Complete Adjuvant, Freund's Incomplete Adjuvant, CpG DNA Vaccine Adjuvant, Poly I:C, Cholera toxin, Cholera toxin B subunit, Liposomes, Saponin Vaccine Adjuvant, DDA Adjuvant, Squalene-based Adjuvants, Etx B subunit Adjuvant, IL-12 Vaccine Adjuvant, LTK63 Vaccine Mutant Adjuvant, TiterMax Gold Adjuvant, Ribi Vaccine Adjuvant, Montanide ISA 720 Adjuvant, Corynebacterium-denved P40 Vaccine Adjuvant, MPL^{™} Adjuvant, AS04, AS02, Lipopolysaccharide Vaccine Adjuvant, Muramyl Dipeptide Adjuvant, CRL1005, Killed Corymb acterium parvum Vaccine Adjuvant, Montanide ISA 51, Bordetella pertussis component Vaccine Adjuvant, Cationic Liposomal Vaccine Adjuvant, Adamantylamide Dipeptide Vaccine Adjuvant, Arlacel A, VSA-3 Adjuvant, Aluminum vaccine adjuvant, Polygen Vaccine Adjuvant, ADJUMER^{™}, Algal Glucan, Bay R1005, Theramide^{®}, Stearyl Tyrosine, Specol, Algammulin, AVRIDINE^{®}, Calcium Phosphate Gel, CTA1-DD gene fusion protein, DOC/Alum Complex, Gamma Inulin, Gerbu Adjuvant, GM-CSF, GMDP, Recombinant hlFN-gamma/Interferon-g, Interleukin- 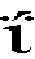 β, Inter leukin-2, Interleukin-7, Sclavo peptide, Rehydragel LV, Rehydragel HP A, Loxoribine, MF59, MTP-PE Liposomes, Murametide, Murapalmitine, D-Murapalmitine, NAGO, Non-Ionic Surfactant Vesicles, PMMA, Protein Cochleates, QS-21, SPT (Antigen Formulation), nanoemulsion vaccine adjuvant, AS03, Quil-A vaccine adjuvant, RC529 vaccine adjuvant, LTR192G Vaccine Adjuvant, E. coli heat-labile toxin, LT, amorphous aluminum hydroxyphosphate sulfate adjuvant, Calcium phosphate vaccine adjuvant, Montanide Incomplete Seppic Adjuvant, Imiquimod, Resiquimod, AF03, Flagellin, Poly(LC), ISCOMATRIX^{®}, Abisco-100 vaccine adjuvant, Albumin-heparin microparticles vaccine adjuvant, AS-2 vaccine adjuvant, B7-2 vaccine adjuvant, DHEA vaccine adjuvant, Immunoliposomes Containing Antibodies to Costimulatory Molecules, SAF-1, Sendai Proteo liposomes, Sendai-containing Lipid Matrices, Threonyl muramyl dipeptide (TMDP), Ty Particles vaccine adjuvant, Bupivacaine vaccine adjuvant, DL-PGL (Polyester poly (DL-lactide-co-glycolide)) vaccine adjuvant, IL-15 vaccine adjuvant, LTK72 vaccine adjuvant, MPL-SE vaccine adjuvant, non-toxic mutant E112K of Cholera Toxin mCT-El 12K, and/or Monophosphoryl lipid A (MPLA), Matrix-S.

In other embodiments, the immunogenic composition of the invention further comprises one or more adjuvants selected from the group comprising selected from the group comprising cationic liposome-DNA complex JVRS-100, aluminum hydroxide vaccine adjuvant, aluminum phosphate, aluminum potassium sulfate, alhydrogel, Freund's Complete Adjuvant, Freund's Incomplete Adjuvant, CpG DNA, Poly I:C, cholera toxin, cholera toxin B subunit, saponin, DDA, Squalene-based adjuvants, Etx B subunit adjuvant, IL-12 vaccine adjuvant, LTK63 vaccine mutant adjuvant, Ribi vaccine adjuvant, Corynebacterium-derived P40 vaccine adjuvant, lipopolysaccharide vaccine adjuvant, muramyl dipeptide adjuvant, killed Corynebacterium parvum vaccine adjuvant, Bordetella pertussis component vaccine adjuvant, cationic Liposomal vaccine adjuvant, Adamantylamide Dipeptide vaccine adjuvant, Aluminum vaccine adjuvant, Algal Glucan, Stearyl Tyrosine, Specol, Algammulin, Calcium Phosphate Gel, CTA1-DD gene fusion protein, DOC/ Alum Complex, Gamma Inulin, Gerbu Adjuvant, GM-CSF, GMDP, Recombinant hlFN-gamma/Interferon-g, Interleukin- 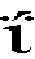 β, Interleukin-2, Interleukin-7, Sclavo peptide, Rehydragel LV, Rehydragel HPA, Loxoribine, MF59, MTP-PE Liposomes, Murametide, Murapalmitine, D-Murapalmitine, NAGO, Non-Ionic Surfactant Vesicles, PMMA, Protein Cochleates, QS-21, SPT (Antigen Formulation), nanoemulsion vaccine adjuvant, Quil-A vaccine adjuvant, RC529 vaccine adjuvant, LTR192G vaccine adjuvant, E. coli heat-labile toxin, LT, amorphous aluminum hydroxyphosphate sulfate adjuvant, Calcium phosphate vaccine adjuvant, Imiquimod, Resiquimod, Flagellin, Poly(LC), Albumin-heparin microparticles vaccine adjuvant, AS-2 vaccine adjuvant, B7-2 vaccine adjuvant, DHEA vaccine adjuvant, Immunoliposomes Containing Antibodies to Costimulatory Molecules, SAF-1, Sendai Proteo liposomes, Sendai-containing Lipid Matrices, Threonyl muramyl dipeptide (TMDP), Ty Particles vaccine adjuvant, Bupivacaine vaccine adjuvant, DL-PGL (Polyester poly (DL-lactide-co-glycolide)) vaccine adjuvant, IL-15 vaccine adjuvant, LTK72 vaccine adjuvant, MPL-SE vaccine adjuvant, non-toxic mutant El 12K of Cholera Toxin mCT-El 12K, Monophosphoryl lipid A (MPLA) and/or Matrix-S.

An aspect of the present invention provides an immunomodulatory compound for use as an adjuvant in vaccination, wherein the immunomodulatory compound is selected from the group consisting of 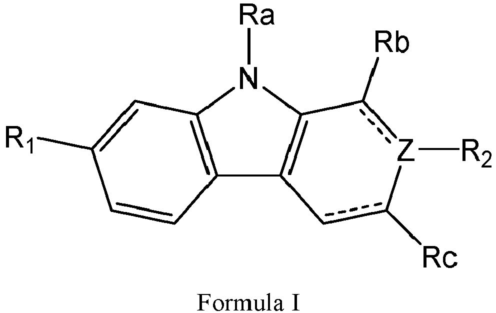 preferably 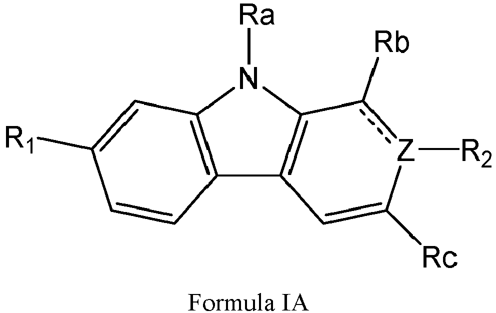 or pharmaceutically acceptable salts thereof
wherein
Z is N or C; preferably Z is N;
R₁ is selected from the group comprising -H, =O, -OH, -S-CH₃, -OCH₃, and -CH₂OH; preferably R₁ is selected from the group comprising -H, =O, -OH, -OCH₃;
R₂ is absent or selected from the group comprising -H, -CH₃, and -CH₂-CH₃; preferably R2 is absent or is -H;
Ra is selected from the group comprising -H, -CH₃ and -(CH₂)n-CH₃, wherein n is 1 to 20 or 1 to 10, preferably n is 1 to 5 or 1 to 3; preferably Ra is -H
Rb is -H or -CH₃;
Rc is -H or -NH₂;
Preferably Z is N and R₂ is absent;
When R₁ is =O, then Z is N and R₂ is -H; 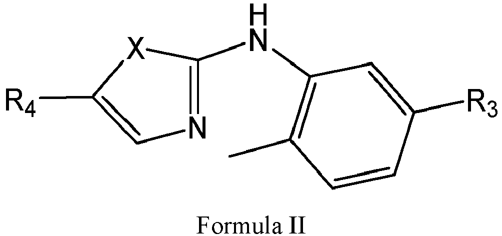 or pharmaceutically acceptable salts thereof
   wherein
   X is selected from the group comprising NH, O, and S;
   R₃ is selected from the group comprising -H, -OH, -OCH₃, -CH₂-OH, and -S-CH₃; preferably R3 is -OCH₃ or -S-CH₃;
   R₄ is selected from the group comprising -CH₃, -CH₂-CH₃, 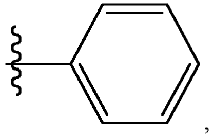 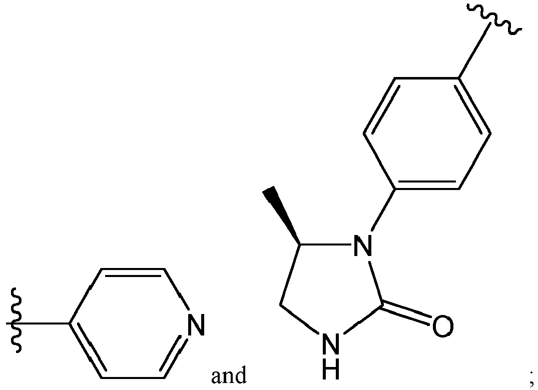 preferably R4 is -CH₃ or -CH₂-CH₃; 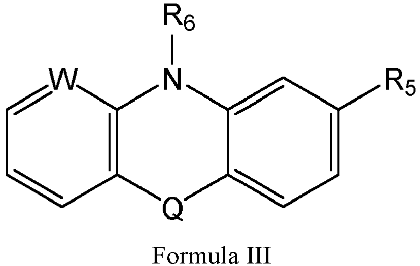 or pharmaceutically acceptable salts thereof
      wherein
      W is N or CH; preferably W is CH;
      Q is selected from the group comprising O, S and NH; preferably Q is S;
      R₅ is selected from the group comprising -H, -OH, -OCH₃, -CH₂OH, and -S-CH₃; preferably R₅ is -H;
      R₆ is selected from the group comprising -H, -CH₃, -CH₂-CH₃, -CH₂-CH₂-N(CH₃)₂, 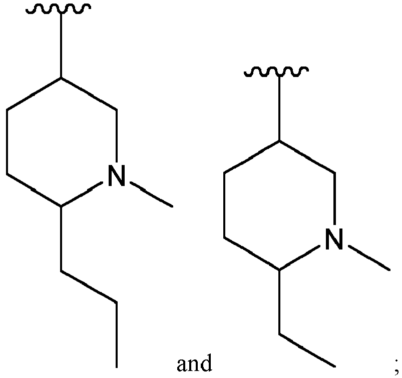 preferably R₆ is -H. 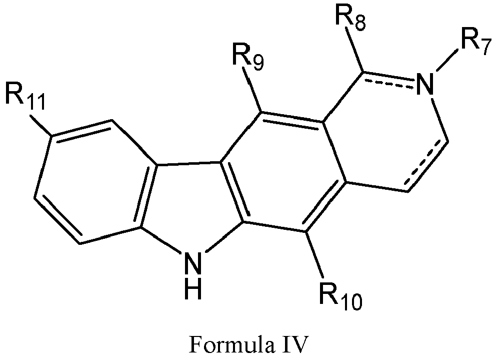
      or pharmaceutically acceptable salts thereof
      wherein
         R7 is absent or selected from the group comprising -H, -CH₃ and -CH₂-CH₃;
         R8 is -H or -CH₃;
         R9 is -H or -CH₃; preferably R9 is -CH₃;
         R10 is -H or CH₃; preferably R10 is -CH₃;
         R11 is selected from the group comprising -H, -OH and -O-CH₃; 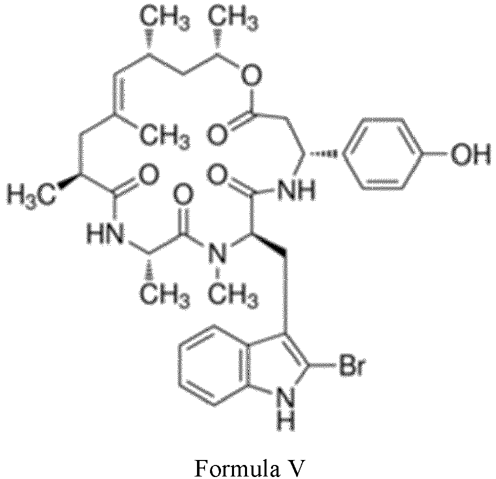
or pharmaceutically acceptable salts thereof; and 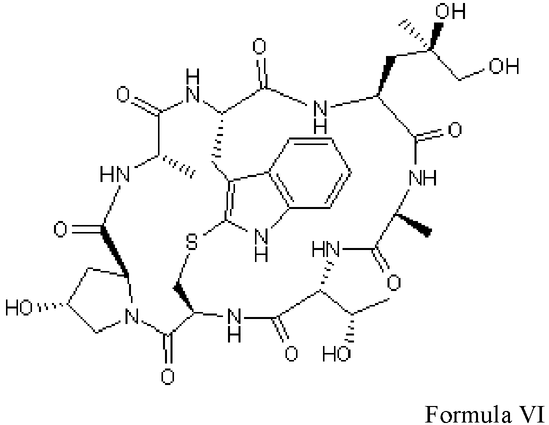 or pharmaceutically acceptable salts thereof.

An aspect of the present invention provides a method of modulating the immune response in a subject comprising administering to the subject an effective amount of the immunogenic composition of the invention or at least one immunomodulatory compound of the invention to improve the immune response in the subject

In an embodiment, the invention provides a method of improving the immune response in a subject comprising administering to the subject an effective amount of the immunogenic composition of the invention or at least one immunomodulatory compound of the invention.

Another embodiment of the invention provides an immunomodulatory compound or an immunogenic composition of the invention for use in the method of improving the immune response in a subject, wherein the immunomodulatory compound is selected from the group consisting of 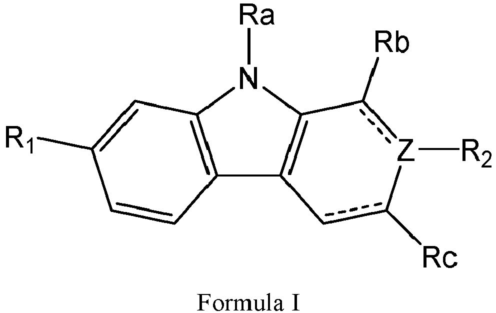 preferably 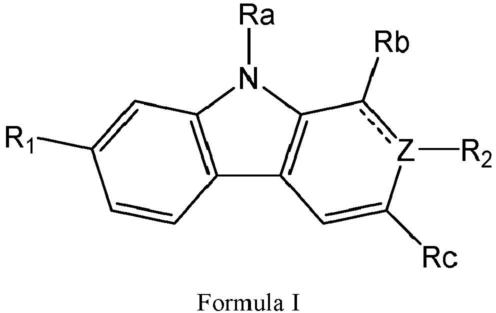 or pharmaceutically acceptable salts thereof
wherein
Z is N or C; preferably Z is N;
R₁ is selected from the group comprising -H, =O, -OH, -S-CH₃, -OCH₃, and -CH₂OH; preferably R₁ is selected from the group comprising -H, =O, -OH, -OCH₃;
R₂ is absent or selected from the group comprising -H, -CH₃, and -CH₂-CH₃; preferably R₂ is absent or is -H;
Ra is selected from the group comprising -H, -CH₃ and -(CH₂)n-CH₃, wherein n is 1 to 20 or 1 to 10, preferably n is 1 to 5 or 1 to 3; preferably Ra is -H;
Rb is -H or -CH₃;
Rc is -H or -NH₂;
Preferably Z is N and R₂ is absent;
When R₁ is =O, then Z is N and R₂ is -H; 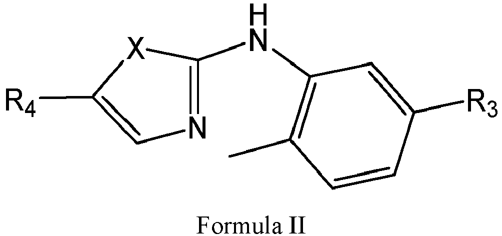 or pharmaceutically acceptable salts thereof
   wherein
   X is selected from the group comprising NH, O, and S;
   R₃ is selected from the group comprising -H, -OH, -OCH₃, -CH₂-OH, and -S-CH₃; preferably R₃ is -OCH₃ or -S-CH₃;
   R₄ is selected from the group comprising -CH₃, -CH₂-CH₃, 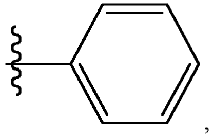 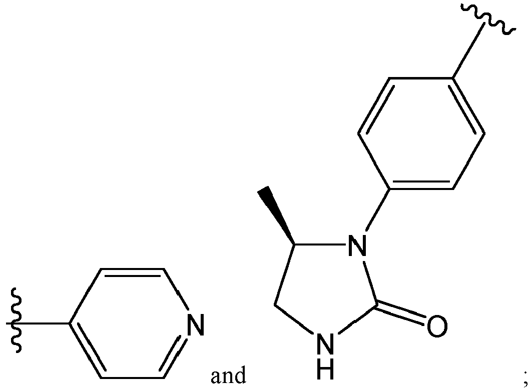 preferably R₄ is -CH₃ or -CH₂-CH₃: 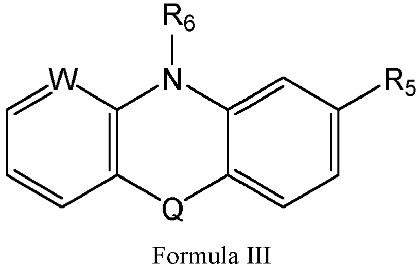 or pharmaceutically acceptable salts thereof
      wherein
      W is N or CH; preferably W is CH;
      Q is selected from the group comprising O, S and NH; preferably Q is S;
      R₅ is selected from the group comprising -H, -OH, -OCH₃, -CH₂OH, and -S-CH₃; preferably R₅ is -H;
      R₆ is selected from the group comprising -H, -CH₃, -CH₂-CH₃, -CH₂-CH₂-N(CH₃)₂, 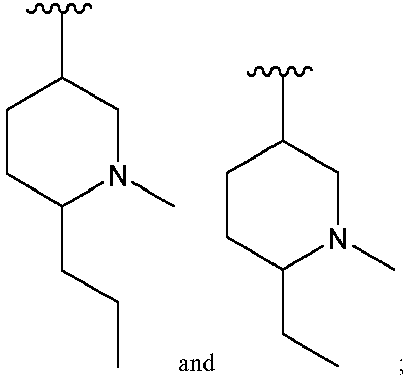 preferably R₆ is -H. 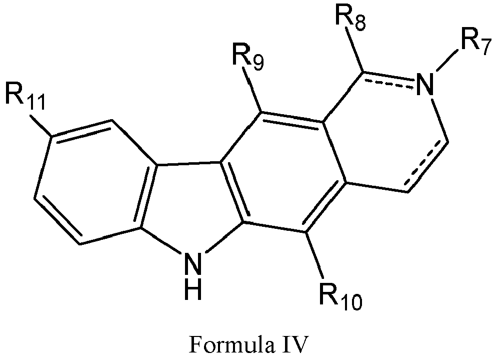 or pharmaceutically acceptable salts thereof
         wherein
         R7 is absent or selected from the group comprising -H, -CH₃ and -CH₂-CH₃; R8 is -H or -CH₃;
         R9 is -H or -CH₃; preferably R9 is -CH₃;
         R10 is -H or CH₃; preferably R10 is -CH₃;
         R11 is selected from the group comprising -H, -OH and -O-CH₃; 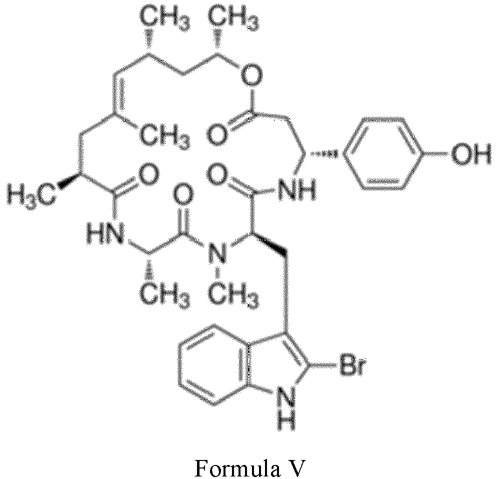
or pharmaceutically acceptable salts thereof; and 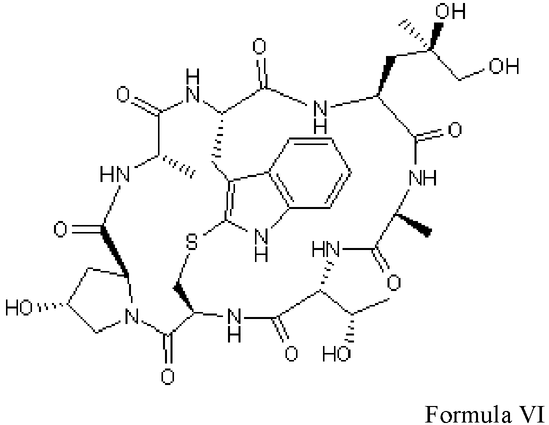 or pharmaceutically acceptable salts thereof.

In some embodiment, the present invention provides a method of modulating the immune response in a subject comprising administering the subject an effective amount of at least one immunomodulatory compound of the invention to modulate and/or improve the immune response in the subject.

In some embodiments, the invention provides an immunogenic composition of the invention or an immunomodulatory compound of the invention for use in the method of modulating the immune response in a subject.

In some other embodiments, the invention provides an immunogenic composition of the invention or an immunomodulatory compound of the invention for use in the method of increasing the immune response in a subject.

In some embodiments, the immunomodulatory compound in the method of the invention is of Formula I, II, III, IV, V or VI. In some other embodiments, the immunomodulatory compound in the method of the invention is of formula I and II. In some other embodiments, the immunomodulatory compound in the method of the invention is of formula I, II and III. In some other embodiments, the immunomodulatory compound in the method of the invention is of formula I, II, III and IV. In some other embodiments, the immunomodulatory compound in the method of the invention is of formula IV, V and VI. In some preferred embodiments, the immunomodulatory compound in the method of the invention is of Formula I or III. In further preferred embodiments, the immunomodulatory compound in the method of the invention is of Formula I.

In some embodiments of the method of the invention, the immunomodulatory compound is selected from the group comprising 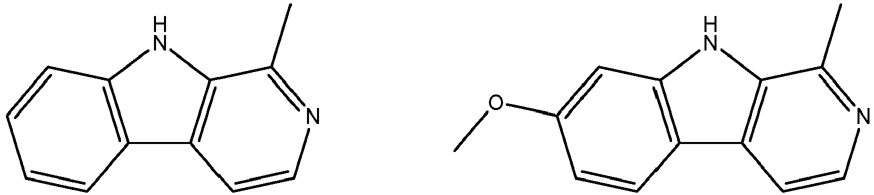 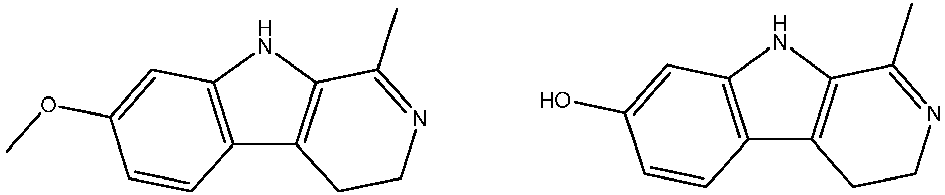 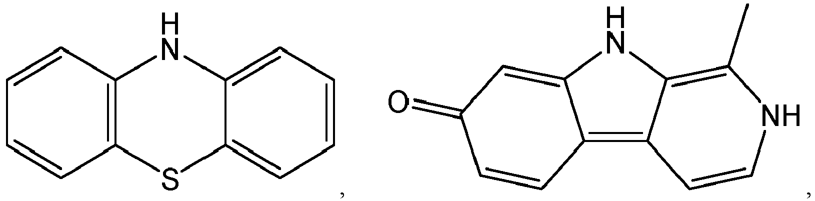 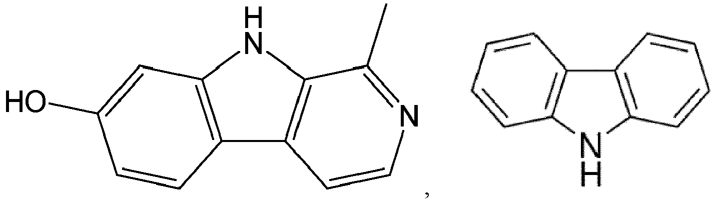 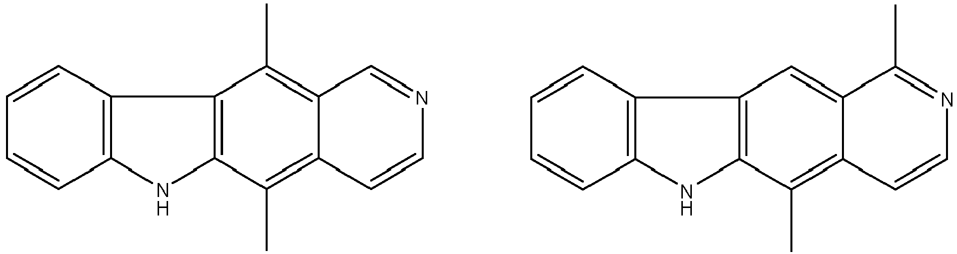 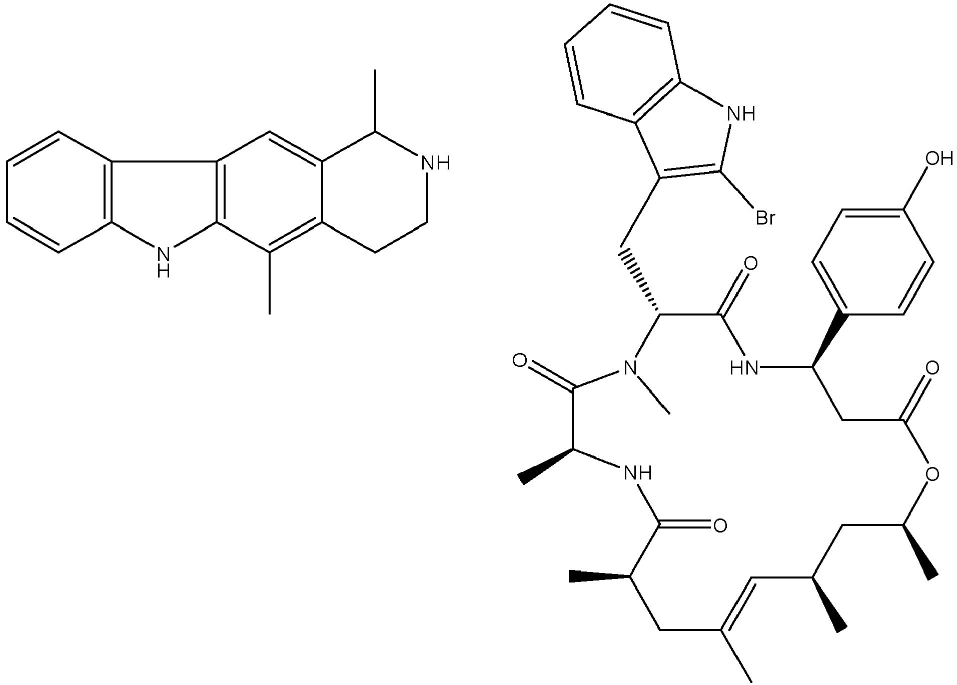 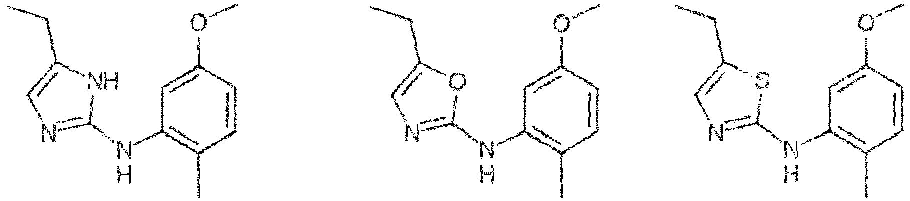 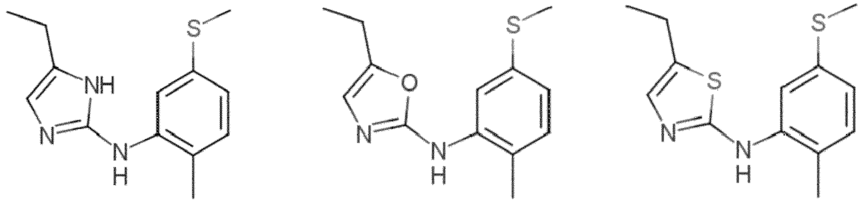 or pharmaceutically acceptable salts thereof.

In an embodiment of the method of the invention, the immunomodulatory compound is selected from the group comprising 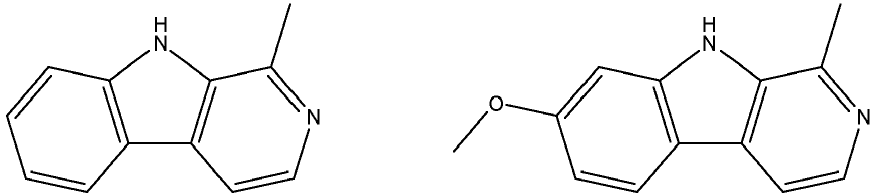 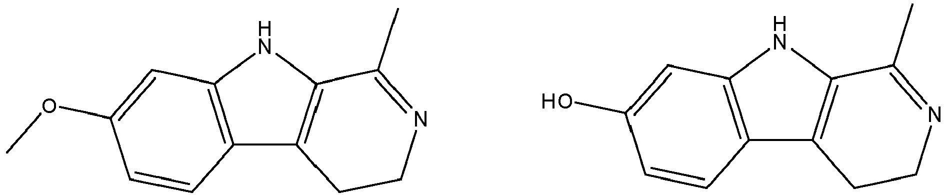 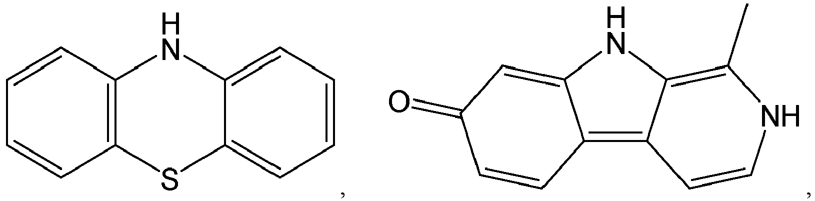 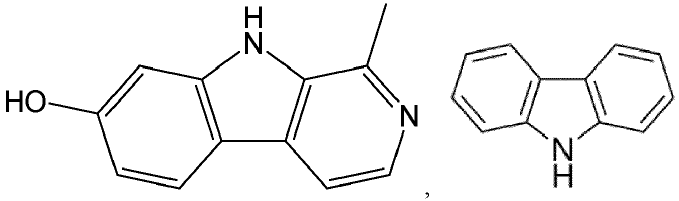 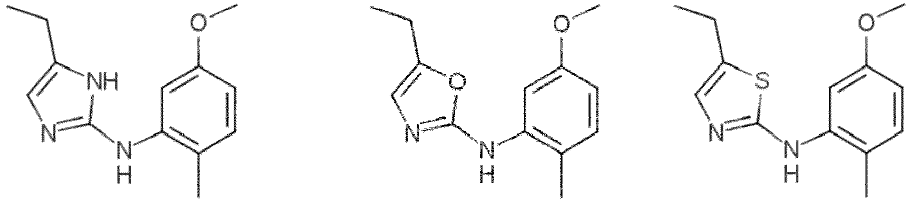 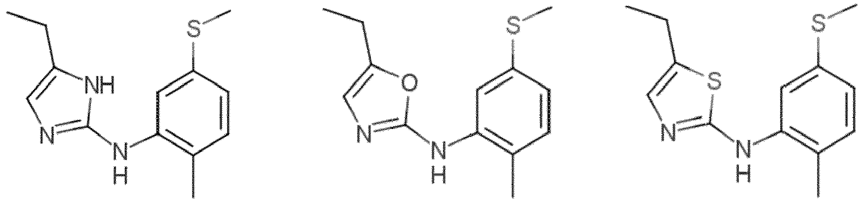 or pharmaceutically acceptable salts thereof.

In a preferred embodiment of the method of the invention, the immunomodulatory compound is selected from the group comprising 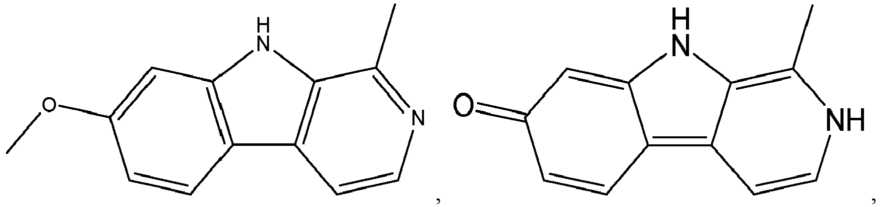 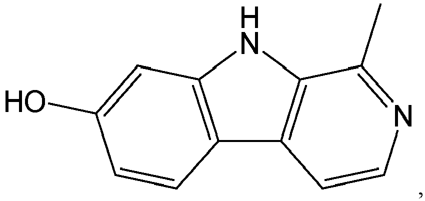 or pharmaceutically acceptable salts thereof.

In another embodiment of the method of the invention, the immunomodulatory compound is selected from the group comprising 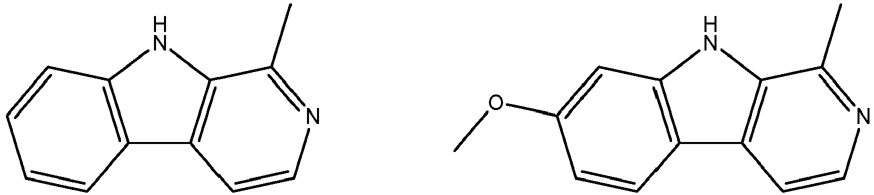 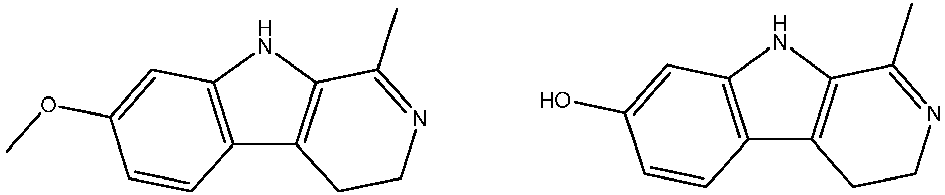 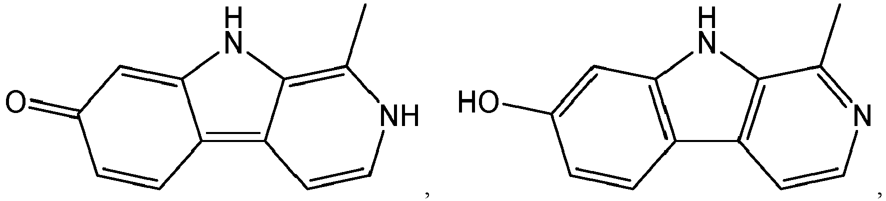 or pharmaceutically acceptable salts thereof
preferably the compound is 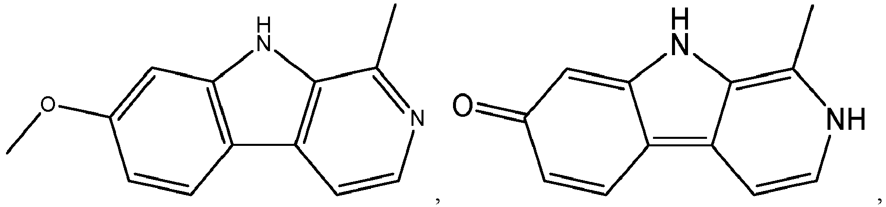 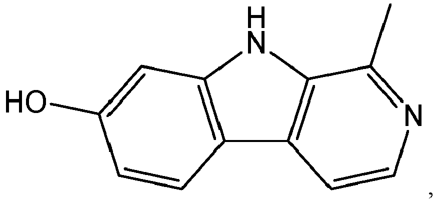 or pharmaceutically acceptable salts thereof.

In another preferred embodiment of the method of the invention, the immunomodulatory compound is selected from the group comprising 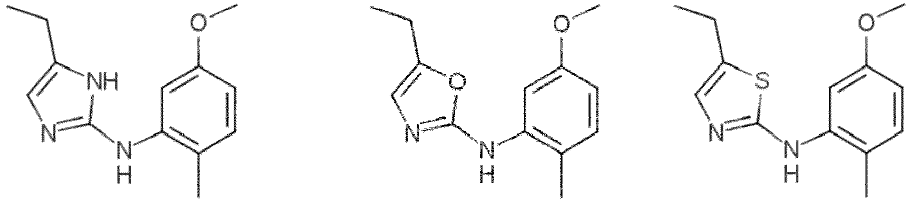 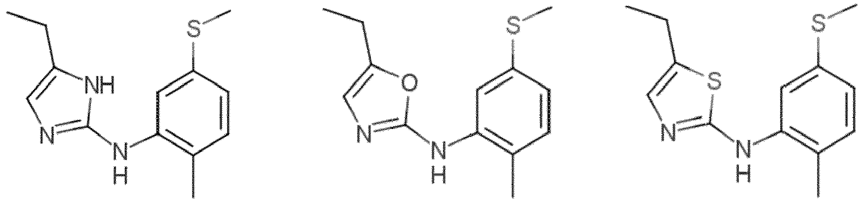 or pharmaceutically acceptable salts thereof.

In some embodiments of the invention, improving the immune response in a subject relates to boosting and/or improving the immune response in a subject during infectious diseases (typically caused by pathogenic microorganisms selected from the group comprising bacteria, viruses, fungi, protozoal pathogens and parasites), autoimmune diseases and/or cancer to facilitate disease resolution or prevention. Thus in some embodiments of the method of improving the immune response in a subject according to the invention, the subject has
a) an infectious disease caused by pathogenic microorganisms selected from the group comprising bacteria, viruses, fungi, protozoal pathogens and parasites,
b) a cancer, preferably the cancer is selected from the group comprising myeloma, prostate cancer, urothelial cancer, breast cancer, melanoma, pancreatic cancer, and/or lung cancer, or
c) an autoimmune disease, preferably the autoimmune disease is selected from the group comprising systemic lupus erythematosus, multiple sclerosis, rheumatic diseases, and Cryopyrin-associated periodic syndrome (CAPS).

In further embodiments of the method of improving the immune response in a subject according to the invention, the method further comprises administering to the subject one or more additional active agents selected from the group comprising
a) antibiotics, anti-bacterial agents, anti-viral agents, antifungal agents, antiparasitic agents, osmotic diuretics, anti-convulsants, anti-pyretics, immunomodulating drugs and/or combinations thereof; or
b) cancer immunotherapeutic agents selected from the group comprising immune checkpoint inhibitor, TCR-T cells, CAR-T cells and combinations thereof. Preferably the immune checkpoint inhibitor is selected from the group comprising nivolumab, pembrolizumab, pidilizumab, ipilimumab, dacarbazine, BMS 936559, atezolizumab, lambrolizumab, avelumab, durvalumab, and/or any combinations thereof.

Another aspect of the invention provides a method for treating and/or preventing an infectious disease comprising administering to the subject an effective amount of the immunogenic composition of the invention or the immunomodulatory compound of the invention, such as the compounds of Formula I, II, III, IV, V and/or VI of the invention, wherein the infectious disease is caused by pathogenic microorganisms selected from the group comprising bacteria, viruses, fungi, protozoal pathogens and parasites.

An embodiment of the invention provides the immunogenic composition of the invention or an immunomodulatory compound of the invention for use in the method of treating and/or preventing an infectious disease, wherein the infectious disease is caused by pathogenic microorganisms selected from the group comprising bacteria, viruses, fungi, protozoal pathogens and parasites.

The preferred mode of administration would depend on the disease being targeted, the type of desired immune response, and if it was being administered as an immunomodulatory compound alone in a combined therapy or as a component of the immunogenic composition, such as a vaccine. The immunomodulatory compound can be administered orally, or as an injectable administered intravenous, intramuscularly, intradermal or subcutaneous.

The adjuvant composition and the immunogenic composition comprising the immunomodulatory compounds of the invention comprise or may comprise a pharmaceutically acceptable excipient and/or carrier, and be formulated for human or veterinary use, and then be administered by various routes. The compositions of the invention can be administered orally, intraperitoneally, intravenously, intramuscularly, subcutaneously, intradermally, or the like. In one embodiment, the compositions of the invention are formulated into an injectable preparation. The injectable preparation can be formulated using an aqueous solution such as saline solution or Ringer's solution, and a non-aqueous solution such as vegetable oil, higher fatty acid ester (for example, ethyl oleate), and alcohols (for example, ethanol, benzylalcoho1, propyleneglycol, or glycerine). Further, the injectable preparation may contain a pharmaceutically acceptable excipient and/or carrier such as a stabilizer to prevent degradation (for example, ascorbic acid, sodium bisulfite, sodium pyrosulfite, BHA, tocopherol, and EDTA), an emulsifier, a buffering agent to adjust pH, and an antimicrobial preservative (for example, phenylmercuric nitrate, thimerosal, benzalkonium chloride, phenol, cresol, and benzylalcohol).

The compositions of the invention are administered in a pharmaceutically effective amount. The phrase "pharmaceutically effective amount" refers to an amount enough to exert the vaccine effects, and further an amount that not to cause an adverse reaction, or serious or excessive immunity response. An exact concentration of administration varies depending on the antigen to be administered and can be easily determined by those skilled in the art, depending on the factors well known in the medical field including patient's age, weight, health condition, sex, and sensitivity to drug, administration route, and administration method, and it can be administered once or multiple times.

In an embodiment, the method further comprises administering to the subject one or more additional active agents selected from the group comprising antibiotics, anti-bacterial agents, anti-viral agents, anti-fungal agents, anti-protozoan agents, anti-parasitic agents, osmotic diuretics, anti-convulsants, anti-pyretics, immunomodulating drugs and combinations thereof.

The additional active agents can be administered simultaneously, for example in a combined unit dose (e.g., providing simultaneous delivery). The additional active agents can also be administered separately or sequentially at a specified time interval, such as, but not limited to, an interval of minutes, hours, days or weeks. Generally, the additional active agents may be concurrently bioavailable, e.g., detectable, in the subject. In some embodiments, the additional active agents may be administered essentially simultaneously, for example two unit dosages administered at the same time, or a combined unit dosage of the two compounds / agents. In other embodiments, the additional active agents may be delivered in separate unit dosages. The additional active agents may be administered in any order, or as one or more preparations that includes two or more agents. In a preferred embodiment, at least one administration of one of the additional active agents, e.g., the first agent, may be made within minutes, one, two, three, or four hours, or even within one or two days of the other agent, e.g., the second agent. In some embodiments, combinations can achieve synergistic results, e.g., greater than additive results, e.g., at least 25, 50, 75, 100, 200, 300, 400, or 500% greater than additive results.

In one specific embodiment, the invention provides a method for treating or preventing a bacterial infection and/or a disease, disorder, or condition associated with a bacterial infection, or a symptom thereof, in a subject, by administering the immunogenic composition of the invention or the immunomodulatory compound of the invention, such as the compounds of Formula I, II, III, IV, V and/or VI of the invention. Said administration may be in combination with an anti-bacterial agent.

Diseases, disorders, or conditions which may be associated with bacterial infections include, but are not limited to one or more of the following: abscesses, actinomycosis, acute prostatitis, aeromonas hydrophila, annual ryegrass toxicity, anthrax, bacillary peliosis, bacteremia, bacterial gastroenteritis, bacterial meningitis, bacterial pneumonia, bacterial vaginosis, bacterium-related cutaneous conditions, bartonellosis, BCG-oma, botryomycosis, botulism, Brazilian purpuric fever, Brodie abscess, brucellosis, Buruli ulcer, campylobacteriosis, caries, Carrion's disease, cat scratch disease, cellulitis, chlamydia infection, cholera, chronic bacterial prostatitis, chronic recurrent multifocal osteomyelitis, clostridial necrotizing enteritis, combined periodontic-endodontic lesions, contagious bovine pleuropneumonia, diphtheria, diphtheritic stomatitis, ehrlichiosis, erysipelas, piglottitis, erysipelas, Fitz-Hugh-Curtis syndrome, flea-borne spotted fever, foot rot (infectious pododermatitis), Garre's sclerosing osteomyelitis, Gonorrhea, Granuloma inguinale, human granulocytic anaplasmosis, human monocytotropic ehrlichiosis, hundred days' cough, impetigo, late congenital syphilitic oculopathy, legionellosis, Lemierre's syndrome, leprosy (Hansen's Disease), leptospirosis, listeriosis, Lyme disease, lymphadenitis, melioidosis, meningococcal disease, meningococcal septicaemia, methicillin-resistant Staphylococcus aureus (MRSA) infection, mycobacterium avium-intracellulare (MAI), mycoplasma pneumonia, necrotizing fasciitis, nocardiosis, noma (cancrum oris or gangrenous stomatitis), omphalitis, orbital cellulitis, osteomyelitis, overwhelming post-splenectomy infection (OPSI), ovine brucellosis, pasteurellosis, periorbital cellulitis, pertussis (whooping cough), plague, pneumococcal pneumonia, Pott disease, proctitis, pseudomonas infection, psittacosis, pyaemia, pyomyositis, Q fever, relapsing fever (typhinia), rheumatic fever, Rocky Mountain spotted fever (RMSF), rickettsiosis, salmonellosis, scarlet fever, sepsis, serratia infection, shigellosis, southern tick-associated rash illness, staphylococcal scalded skin syndrome, streptococcal pharyngitis, swimming pool granuloma, swine brucellosis, syphilis, syphilitic aortitis, tetanus, toxic shock syndrome (TSS), trachoma, trench fever, tropical ulcer, tuberculosis, tularemia, typhoid fever, typhus, urogenital tuberculosis, urinary tract infections, vancomycin-resistant Staphylococcus aureus infection, Waterhouse-Friderichsen syndrome, pseudotuberculosis (Yersinia) disease, and yersiniosis. Other diseases, disorders, and/or conditions associated with bacterial infections can include, for example, Alzheimer's disease, anorexia nervosa, asthma, atherosclerosis, attention deficit hyperactivity disorder, autism, autoimmune diseases, bipolar disorder, cancer (e.g., colorectal cancer, gallbladder cancer, lung cancer, pancreatic cancer, and stomach cancer), chronic fatigue syndrome, chronic obstructive pulmonary disease, Crohn's disease, coronary heart disease, dementia, depression, Guillain-Barre syndrome, metabolic syndrome, multiple sclerosis, myocardial infarction, obesity, obsessive-compulsive disorder, panic disorder, psoriasis, rheumatoid arthritis, sarcoidosis, schizophrenia, stroke, thromboangiitis obliterans (Buerger's disease), and Tourette syndrome.

The bacterium described herein can be a Gram-positive bacterium or a Gram-negative bacterium. Bacterial pathogens include, but are not limited to, Acinetobacter baumannii, Bacillus anthracis, Bacillus subtilis, Bordetella pertussis, Borrelia burgdorferi, Brucella abortus, Brucella canis, Brucella melitensis, Brucella suis, Burkholderia, Campylobacter jejuni, Chlamydia pneumoniae, Chlamydia trachomatis, Chlamydophila psittaci, Clostridium botulinum, Clostridium difficile, Clostridium perfringens, Clostridium tetani, coagulase Negative Staphylococcus, Corynebacterium diphtheria, Enterococcus faecalis, Enterococcus faecium, Escherichia coli, enterotoxigenic Escherichia coli (ETEC), enteropathogenic E. coli, E. coli 0157:H7, Enterobacter sp., Francisella tularensis, Haemophilus influenzae, Helicobacter pylori, Klebsiella pneumoniae, Legionella pneumophila, Leptospira interrogans, Listeria monocytogenes, Moraxella catarralis, Mycobacterium leprae, Mycobacterium tuberculosis, Mycoplasma pneumoniae, Neisseria gonorrhoeae, Neisseria meningitides, Preteus mirabilis, Proteus sps., Pseudomonas aeruginosa, Rickettsia rickettsii, Salmonella typhi, Salmonella typhimurium, Serratia marcesens, Shigella flexneri, Shigella sonnei, Staphylococcus aureus, Staphylococcus epidermidis, Staphylococcus saprophyticus, Streptococcus agalactiae, Streptococcus mutans, Streptococcus pneumoniae, Streptococcus pyogenes, Treponema pallidum, Vibrio cholerae, and Yersinia pestis. Bacterial pathogens may also include bacteria that cause resistant bacterial infections, for example, clindamycin-resistant Clostridium difficile, fluoroquinolon-resistant Clostridium difficile, methicillin-resistant Staphylococcus aureus (MRSA), multidrug-resistant Enterococcus faecalis, multidrug-resistant Enterococcus faecium, multidrug-resistance Pseudomonas aeruginosa, multidrug-resistant Acinetobacter baumannii, and vancomycin-resistant Staphylococcus aureus (VRSA).

In one embodiment, the immunogenic composition of the invention or the immunomodulatory compound of the invention may be administered in conjunction with one or more antibiotics. These include, but are not limited to Aknilox, Ambisome, Amoxycillin, Ampicillin, Augmentin, Avelox, Azithromycin, Bactroban, Betadine, Betnovate, Blephamide, Cefaclor, Cefadroxil, Cefdinir, Cefepime, Cefix, Cefixime, Cefoxitin, Cefpodoxime, Cefprozil, Cefuroxime, Cefzil, Cephalexin, Cephazolin, Ceptaz, Chloramphenicol, Chlorhexidine, Chloromycetin, Chlorsig, Ciprofloxacin, Clarithromycin, Clindagel, Clindamycin, Clindatech, Cloxacillin, Colistin, Co-trimoxazole, Demeclocycline, Diclocil, Dicloxacillin, Doxycycline, Duricef, Erythromycin, Flamazine, Floxin, Framycetin, Fucidin, Furadantin, Fusidic, Gatifloxacin, Gemifloxacin, Gemifloxacin, Ilosone, Iodine, Levaquin, Levofloxacin, Lomefloxacin, Maxaquin, Mefoxin, Meronem, Minocycline, Moxifloxacin, Myambutol, Mycostatin, Neosporin, Netromycin, Nitrofurantoin, Norfloxacin, Norilet, Ofloxacin, Omnicef, Ospamox, Oxytetracycline, Paraxin, Penicillin, Pneumovax, Polyfax, Povidone, Rifadin, Rifampin, Rifaximin, Rifinah, Rimactane, Rocephin, Roxithromycin, Seromycin, Soframycin, Sparfloxacin, Staphlex, Targocid, Tetracycline, Tetradox, Tetralysal, Tobramycin, Trecator, Tygacil, Vancocin, Velosef, Vibramycin, Xifaxan, Zagam, Zitrotek, Zoderm, Zymar, and Zyvox.

In another embodiment, provided are methods for treating or preventing a viral infection and/or a disease, disorder, or condition associated with a viral infection, or a symptom thereof, in a subject, by administering the immunogenic composition of the invention or the immunomodulatory compound of the invention, such as the compounds of Formula I, II, III, IV, V and/or VI of the invention.

Diseases, disorders, or conditions associated with viral infections include, but are not limited to, acute febrile pharyngitis, pharyngoconjunctival fever, epidemic keratoconjunctivitis, infantile gastroenteritis, Coxsackie infections, infectious mononucleosis, Burkitt lymphoma, acute hepatitis, chronic hepatitis, hepatic cirrhosis, hepatocellular carcinoma, primary HSV-1 infection (e.g., gingivostomatitis in children, tonsillitis and pharyngitis in adults, keratoconjunctivitis), latent HSV-1 infection (e.g., herpes labialis and cold sores), primary HSV-2 infection, latent HSV-2 infection, aseptic meningitis, infectious mononucleosis, Cytomegalic inclusion disease, Kaposi sarcoma, multicentric Castleman disease, primary effusion lymphoma, AIDS, influenza, Reye syndrome, measles, postinfectious encephalomyelitis, Mumps, hyperplastic epithelial lesions (e.g., common, flat, plantar and anogenital warts, laryngeal papillomas, epidermodysplasia verruciformis), cervical carcinoma, squamous cell carcinomas, croup, pneumonia, bronchiolitis, common cold, Poliomyelitis, Rabies, bronchiolitis, pneumonia, influenza-like syndrome, severe bronchiolitis with pneumonia, German measles, congenital rubella, SARs, Coronavirus infections, Yellow fever, Dengue, Varicella, and herpes zoster.

Viral pathogens include, but are not limited to, adenovirus, coxsackievirus, dengue virus, encephalitis virus, Epstein-Barr virus, hepatitis A virus, hepatitis B virus, hepatitis C virus, herpes simplex virus type 1, herpes simplex virus type 2, cytomegalovirus, human herpesvirus type 8, human immunodeficiency virus, influenza virus, measles virus, mumps virus, human papillomavirus, parainfluenza virus, poliovirus, rabies virus, respiratory syncytial virus, rubella virus, varicella-zoster virus, West Nile virus, and yellow fever virus. Viral pathogens may also include viruses that cause resistant viral infections.

In one embodiment, the immunogenic composition of the invention or the immunomodulatory compound of the invention may be administered in conjunction with one or more antiviral agents. Exemplary anti-viral agents include, but are not limited to, abacavir (ZIAGEN^{®}), abacavir/lamivudine/zidovudine (trizivir^{®}), aciclovir or acyclovir (CYCLOVIR^{®}, HERPEX^{®}, ACIVIR^{®}, ACIVIRAX^{®}, ZOVIRAX^{®}, ZOVIR^{®}), adefovir (Preveon^{®}, Hepsera^{®}), amantadine (SYMMETREL^{®}), amprenavir (AGENERASE^{®}), ampligen, arbidol, atazanavir (REYATAZ^{®}), boceprevir, cidofovir, darunavir (PREZISTA^{®}), delavirdine (RESCRIPTOR^{®}), didanosine (VIDEX^{®}), docosanol (ABREVA^{®}), edoxudine, efavirenz (SUSTIVA^{®}, STOCRIN^{®}), emtricitabine (EMTRT A^{®}), emtricitabine/tenofovir/efavirenz (ATRIP LA^{®}), enfuvirtide (FUZEON^{®}), entecavir (BARACLUDE^{®}, ENTAVIR^{®}), famciclovir (FAMVIR^{®}), fomivirsen (VITRAVENE^{®}), fosamprenavir (LEXIVA^{®}, TELZIR^{®}), foscarnet (FOSCAVIR^{®}), fosfonet, ganciclovir (CYTOVENE^{®}, CYMEVENE^{®}, VITRASERT^{®}), GS 9137 (ELVITEGRAVIR^{®}), imiquimod (ALDARA^{®}, ZYCLARA^{®}, BESELNA^{®}), indinavir (CRIXIVAN^{®}), inosine, inosine pranobex (IMUNOVIR^{®}), interferon type I, interferon type II, interferon type III, kutapressin (NEXAVIR^{®}), lamivudine (ZEFFIX^{®}, HEPTOVIR^{®}, EPIVIR^{®}), lamivudine/zidovudine (COMBIVIR^{®}), lopinavir, loviride, maraviroc (SELZENTRY^{®}, CELSENTRI^{®}), methisazone, MK-2048, moroxydine, nelfmavir (VIRACEPT^{®}), nevirapine (VIRAMUNE^{®}), oseltamivir (TAMIFLU^{®}), peginterferon alfa-2a (PEGASYS^{®}), penciclovir (DENAVIR^{®}), peramivir, pleconaril, podophyllotoxin (CONDYLOX^{®}), raltegravir (ISENTRESS^{®}), ribavirin (COPEGUs^{®}, REBETOL^{®}, RIBASPHERE^{®}, VILONA^{®} AND VIRAZOLE^{®}), rimantadine (FLUMADINE^{®}), ritonavir (NORVIR^{®}), pyramidine, saquinavir (INVIRASE^{®}, FORTOVASE^{®}), stavudine, tea tree oil (melaleuca oil), tenofovir (VIREAD^{®}), tenofovir/emtricitabine (TRUVADA^{®}), tipranavir (APTIVUS^{®}), trifluridine (VIROPTIC^{®}), tromantadine (VIRU-MERZ^{®}), valaciclovir (VALTREX^{®}), valganciclovir (VALCYTE^{®}), vicriviroc, vidarabine, viramidine, zalcitabine, zanamivir (RELENZA^{®}), and zidovudine (azidothymidine (AZT), RETROVIR^{®}, RETROVIS^{®}).

In another embodiment, provided are methods for treating or preventing a fungal infection and/or a disease, disorder, or condition associated with a fungal infection, or a symptom thereof, in a subject, by administering the immunogenic composition of the invention or the immunomodulatory compound of the invention, such as the compounds of Formula I, II, III, IV, V and/or VI of the invention.

Diseases, disorders, or conditions associated with fungal infections include, but are not limited to, aspergilloses, blastomycosis, candidasis, coccidioidomycosis, cryptococcosis, histoplasmosis, mycetomas, paracoccidioidomycosis, and tinea pedis. Furthermore, persons with immuno-deficiencies are particularly susceptible to disease by fungal genera such as Aspergillus, Candida, Cryptoccocus, Histoplasma, and Pneumocystis. Other fungi can attack eyes, nails, hair, and especially skin, the so-called dermatophytic fungi and keratinophilic fungi, and cause a variety of conditions, of which ringworms such as athlete's foot are common. Fungal spores are also a major cause of allergies, and a wide range of fungi from different taxonomic groups can evoke allergic reactions in some people.

Fungal pathogens include, but are not limited to, Ascomycota (e.g., Fusarium oxysporum, Pneumocystis jirovecii, Aspergillus spp., Coccidioides immitis/posadasii, Candida albicans), Basidiomycota (e.g., Filobasidiella neoformans, Trichosporon), Microsporidia (e.g., Encephalitozoon cuniculi, Enterocytozoon bieneusi), and Mucoromycotina (e.g., Mucor circinelloides, Rhizopus oryzae, Lichtheimia corymbifera).

In one embodiment, the immunogenic composition of the invention or the immunomodulatory compound of the invention may be administered in conjunction with one or more anti-fungal agents. Exemplary anti-fungal agents include, but are not limited to, polyene antifungals {e.g., natamycin, rimocidin, filipin, nystatin, amphotericin B, candicin, hamycin), imidazole antifungals (e.g., miconazole (MICATIN^{®}, DAKTARIN^{®}), ketoconazole (NIZORAL^{®}, FUNGORAL^{®}, SEBIZOLE^{®}), clotrimazole (LOTRIMIN^{®}, LOTRIMIN^{®} AF, CANESTEN^{®}), econazole, omoconazole, bifonazole, butoconazole, fenticonazole, isoconazole, oxiconazole, sertaconazole (ERTACZO^{®}), sulconazole, tioconazole), triazole antifungals (e.g., albaconazole fluconazole, itraconazole, isavuconazole, ravuconazole, posaconazole, voriconazole, terconazole), thiazole antifungals (e.g., abafungin), allylamines (e.g., terbinafine (LAMISIL^{®}), naftifme (NAFTIN^{®}), butenafme (LOTRIMIN^{®} Ultra)), echinocandins (e.g., anidulafungin, caspofungin, micafungin), and others (e.g., polygodial, benzoic acid, ciclopirox, tolnaftate (TINACTIN^{®}, DESENEX^{®}, AFTATE^{®}), undecylenic acid, flucytosine or 5-fluorocytosine, griseofulvin, haloprogin, sodium bicarbonate, allicin). Conditions associated with protozoal infection

In another embodiment, provided are methods for treating or preventing a protozoan infection and/or a disease, disorder, or condition associated with a protozoan infection, or a symptom thereof, in a subject, by administering the immunogenic composition of the invention or the immunomodulatory compound of the invention, such as the compounds of Formula I, II, III, IV, V and/or VI of the invention.

Diseases, disorders, or conditions associated with protozoal infections include, but are not limited to, amoebiasis, giardiasis, trichomoniasis, African Sleeping Sickness, American Sleeping Sickness, cutaneous and visceral leishmaniasis, Chagas disease, balantidiasis, toxoplasmosis, malaria, acanthamoeba keratitis, and babesiosis, echinococcosis, schistosomiasis, lymphatic filiariasis., toxoplasmosis.

Protozoal pathogens include, but are not limited to, Entamoeba histolytica, Giardia lambila, Trichomonas vaginalis, Trypanosoma brucei, T. cruzi, Leishmania donovani, Balantidium coli, Toxoplasma gondii, Plasmodium spp., and Babesia microti.

In one embodiment, the immunogenic composition of the invention or the immunomodulatory compound of the invention may be administered in conjunction with one or more anti-protozoan agents. Exemplary anti-protozoal agents include, but are not limited to, eflomithine, furazolidone (FUROXONE^{®}, DEPENDAL-M^{®}), melarsoprol, metronidazole (FLAGYL^{®}), ornidazole, paromomycin sulfate (HUMATIN^{®}), pentamidine, pyrimethamine (DARAPRIM^{®}), and imidazole (TINDAMAX^{®}, FASIGYN^{®}).

In another embodiment, provided are methods for treating or preventing a parasitic infection and/or a disease, disorder, or condition associated with a parasitic infection, or a symptom thereof, in a subject, by administering the immunogenic composition of the invention or the immunomodulatory compound of the invention, such as the compounds of Formula I, II, III, IV, V and/or VI of the invention.

Diseases, disorders, or conditions associated with parasitic infections include, but are not limited to, acanthamoeba keratitis, amoebiasis, ascariasis, babesiosis, balantidiasis, baylisascariasis, chagas disease, clonorchiasis, cochliomyia, cryptosporidiosis, diphyllobothriasis, dracunculiasis, echinococcosis, elephantiasis, enterobiasis, fascioliasis, fasciolopsiasis, filariasis, giardiasis, gnathostomiasis, hymenolepiasis, isosporiasis, katayama fever, leishmaniasis, lyme disease, malaria, metagonimiasis, myiasis, onchocerciasis, pediculosis, scabies, schistosomiasis, sleeping sickness, strongyloidiasis, taeniasis, toxocariasis, toxoplasmosis, trichinosis, and trichuriasis.

Parasitic pathogens include, but are not limited to, Acanthamoeba, Anisakis, Ascaris lumbricoides, botfly, Balantidium coli, bedbug, Cestoda, chiggers, Cochliomyia hominivorax, Entamoeba histolytica, Fasciola hepatica, Giardia lamblia, hookworm, Leishmania, Linguatula serrata, liver fluke, Loa loa, Paragonimus, pinworm, Plasmodium falciparum, Schistosoma, Strongyloides stercoralis, mite, tapeworm, Toxoplasma gondii, Trypanosoma, whipworm, Wuchereria bancrofti, Schistosomiasis.

In one embodiment, the immunogenic composition of the invention or the immunomodulatory compound of the invention may be administered in conjunction with one or more anti-parasitic agents. Exemplary anti-parasitic agents include, but are not limited to, antinematodes (e.g., mebendazole, pyrantel pamoate, thiabendazole, diethylcarbamazine, ivermectin), anticestodes (e.g., niclosamide, praziquantel, albendazole), antitrematodes (e.g., praziquantel), antiamoebics (e.g., rifampin, amphotericin B), and antiprotozoals (e.g., melarsoprol, eflomithine, metronidazole, imidazole).anti-leishmanials of (pentavalent antimonials, amphotericin B and liposomal amphotericin B, miltefosine, paromycin,

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended claims, and all changes which come within the meaning and range of equivalency are intended to be embraced therein.

The foregoing description will be more fully understood with reference to the following Examples. Such Examples, are, however, exemplary of methods of practising the present invention and are not intended to limit the application and the scope of the invention.

### EXAMPLES

### Example 1

To demonstrate the capacity of the immunomodulatory compounds of the invention to effect beneficial changes to DC maturation and antigen-presentation, the OVA or Eα model antigens were used for which specific antibodies exist to detect specific peptide:MHC complexes on the cell surface. Specifically, these complexes were recognized using fluorochrome-labelled monoclonal antibodies of Y-Ae for I-Ab: Eα₅₅₋₆₈ complexes and 25-D1.16 for H2-Kb: OVA₂₅₇₋₂₆₄ complexes. In all experiments, BMDC were differentiated *in vitro* from bone marrow precursor cells of C57BL/6 mice using GM-CSF and IL-4 at 250 and 5 UI/ml, respectively. A hydrochloride salt forms of ACB1801 and ACB1804 (harmine HCl - ACB1801s and harmol HCl - ACB 1804s) were used for experimentation and a vehicle control was included.

The first experiment evaluated the capacity of ACB 1801s and ACB 1804s to effect changes in BMDC stimulated with a combination of OVA₂₅₇₋₂₆₄ peptide (10µM) and Lipopolysaccharide (LPS) derived from *E.coli* 0111:B4. LPS (100ng/ml) was used in this instance as a model Toll-like Receptor ligand for a pathogen and a surrogate for the licensed TLR-specific adjuvant-MPLA. ACB1801s and ACB1804s increased the overall expression of surface MHC-II expression, concomitant with increased expression of CD80/ CD86 co-stimulatory markers (Figure 1A and B). ACB1801s and ACB1804s treatment also increased the relative expression of stable MHC-I (H2-Kb):OVA peptide complexes over that of the total H2-Kb complexes on the cell surface after 24hours of exposure (Figure 1C). ACB1801s reduced the high levels of pro-inflammatory cytokines (IL-6, and TNF) after 24 hours, yet did not completely abrogate secretion (Figure 1D). Similar results were obtained using ACB1804s (data not shown). This reduction of cytokine production suggests that the immunomodulatory capacity does not act through TLR-ligation as stimulation of antigen presenting immune cells by TLR-ligands resulted in increased cytokine production and combinations of multiple TLRs at the same time would result in levels above that of the conditions tested with LPS alone and in the presence of antigenic peptides.

The second experiment was performed investigating the effect of ACB1801s on the presentation of antigenic peptides in LPS-matured DC. In this experiment BMDc were differentiated with 100ng/ml of ultrapure LPS for 36h in the absence of any immunomodulatory compound of the invention. The cells were pretreated with ACB1801s for 1 hour prior to the pulsing the DC with the Eα₅₅₋₆₈ peptide for 3 hours. The BMDC were analyzed after 24hours for the surface expression of MHC-II (I-Ab): Eα₅₅₋₆₈ complexes on the cell surface in live, CD11c+MHC-II (I-Ab)^{high} cells. ACB1801s pre-treatment elicited a significant elevation of the Median Fluorescence Intensity (MFI) of I-Ab: Eα₅₅₋₆₈ specific complexes on the cell as compared to vehicle controls (1742.33±15.62 versus 1553.33±54.93; p=0.03 using an unpaired student's t-test).

As the overall capacity for antigen-presentation could also be improved under these conditions it demonstrates that mechanisms related to the processing and transport of the antigens to the cell-surface are involved in addition to the effects given by the differences in the maturation state of the DC as demonstrated in Figure 1.

The effect of ACB1801s and ACB1804s was established on endocytosis and the subsequent presentation of antigens onto the cell surface of immature DC (determined by gating on CD11c+MHC-II^{int}). In this experiment the cells derived from 3 individual mice were pretreated for 3hours with ACB1801s (20µM) or vehicle prior to the administration of OVA-AF647 at 2µg/ml. It was clearly shown that ACB1801s and ACB1804s could clearly improve the overall uptake of OVA protein. Although the percentage of OVA-AF647+ cells remained the same at 72.37+/-0.94, 73.867+/-0.751 vs 71.00+/-0.058 for ACB1801s or ACB1804s treated as compared to the vehicle control, within this positive population there was a significant increase in the amount of protein taken up- as given by a change in Median Fluorescence Intensity of 6725.67+/- 41.450 and 6628+/- 98.475 as compared to vehicle controls of 5533.33+/-60.26 (P≤0.0001 for each case).

ACB1801s and ACB1804s could also impact the ability to present the H2-Kb:OVA₂₅₇₋₂₆₄ complexes on the cell surface in iBMDc after administration of OVA protein at 2µg/ml where there was an overall significant increase in the MFI of the DC that were H2:Kb:OVA positive, as assessed using the specific antibody. The results were 3662.5+/- 41.5, 3453.33 +/- 57.8 and 2997.33+/-15.30 for ACB1801s and ACB1804s-treated vs Vehicle control (p=0.016 and p=0.0651, respectively).

These results demonstrate that immunomodulatory compounds of the invention serve to amplify the capacity to antigen-presenting cells to express antigenic peptides in the context of MHC I and II and enhances the maturation of dendritic cells through the preferential increased expression of CD86 and slightly for CD80.

All together these factors demonstrate that immunomodulatory compounds of the invention function as an adjuvant serving to improve immunological synapse formation and strength through which signals required for antigen presentation to be provided to T cells. Strong signals elicited by the T cell receptor and amplified by increased co-stimulatory molecule expression directly influences the polarization T helper-dependent immune responses and cross-presentation. All the while increasing the sensitivity of the T cells to respond to cytokines by upregulating cytokine receptors and reducing the impact of pathological cytokine production.

### Example 2

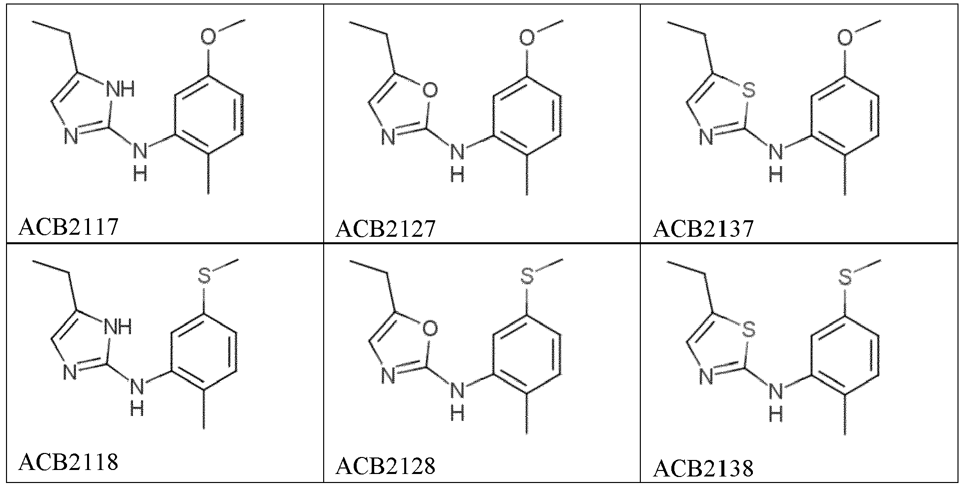

The compounds ACB2117, ACB2127, ACB2137, ACB2118, ACB2128 and ACB2138 are tested in a similar strategy to the studies for ACB1801 and ACB1804 (see Example 1). Specifically, the study evaluates the impact on the immune response in DC with a focus on different aspects of the immunological synapse on the side of the dendritic cells for antigen presentation with or without the presence of an adjuvant, such as a TLR-ligand or a pathogen. The impact of the immunomodulatory compounds are assessed in terms of the ability to effect changes to endocytosis and antigen processing. Subsequently, the immunomodulatory compounds ACB2117, ACB2127, ACB2137, ACB2118, ACB2128 and ACB2138 are assessed for their ability to improve MHC: Antigen complex formation at the cell surface as a function of the total expression of MHC expression (This includes antigen presentation via both MHC-I and MHC-II). The study includes evaluation of the impact of the immunomodulatory compounds on immature, activated and mature dendritic cells. Expression of surface markers involved in disease resolution and protection are analyzed, including those of co-stimulatory molecules of CD86, and CD80 that provide signal 2 and the impact on cytokine secretion analyzed.

## Claims

1. An immunomodulatory compound 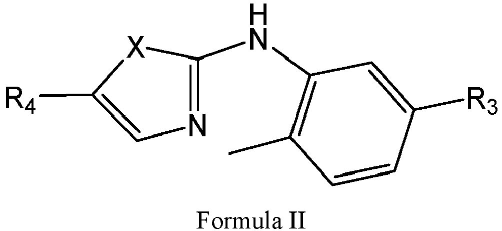 or pharmaceutically acceptable salts thereof
wherein
X is selected from the group comprising NH, O, and S;
R₃ is selected from the group comprising -H, -OH, -OCH₃, -CH₂-OH, and -S-CH₃;
R₄ is selected from the group comprising -CH₃, -CH₂-CH₃,

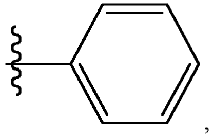

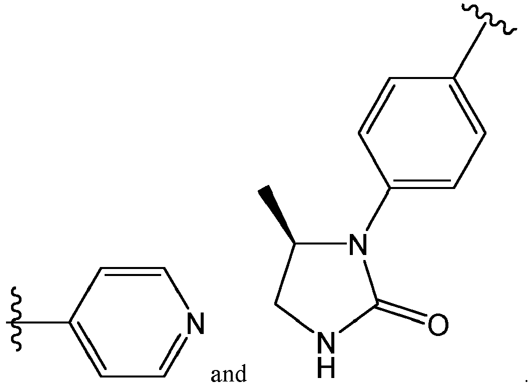

2. The immunomodulatory compound of claim 1, wherein R₃ is -OCH₃.

3. The immunomodulatory compounds of claim 1 or claim 2, wherein R4 is -CH₃.

4. The immunomodulatory compound of claim 1 selected from the group comprising 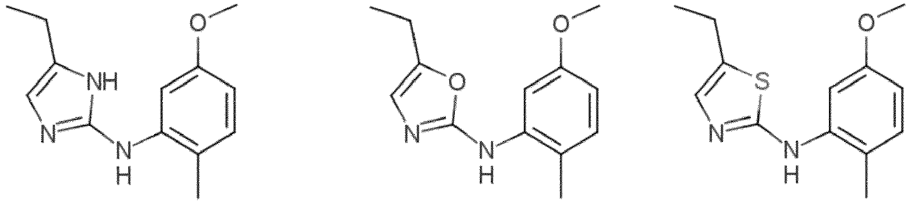 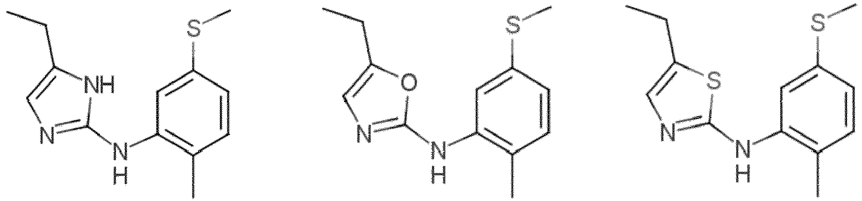 or pharmaceutically acceptable salts thereof.

5. An adjuvant composition comprising the immunomodulatory compound of any one of claims 1-4 and at least one pharmaceutically acceptable excipient and/or carrier.

6. An immunogenic composition comprising an antigen and the immunomodulatory compound of any one of claims 1-4.

7. The immunogenic composition of claim 6, wherein the antigen is selected from the group comprising peptides, proteins, polysaccharides, saccharides, lipids, nucleic acids, or combinations thereof.

8. The immunogenic compositions of any one of claims 6-7, further comprising one or more adjuvants selected from the group comprising cationic liposome-DNA complex JVRS-100, aluminum hydroxide vaccine adjuvant, aluminum phosphate, aluminum potassium sulfate, alhydrogel, Freund's Complete Adjuvant, Freund's Incomplete Adjuvant, CpG DNA, Poly I:C, cholera toxin, cholera toxin B subunit, saponin, DDA, Squalene-based adjuvants, Etx B subunit adjuvant, IL-12 vaccine adjuvant, LTK63 vaccine mutant adjuvant, Ribi vaccine adjuvant, Corynebacterium-derived P40 vaccine adjuvant, lipopolysaccharide vaccine adjuvant, muramyl dipeptide adjuvant, killed Corynebacterium parvum vaccine adjuvant, Bordetella pertussis component vaccine adjuvant, cationic Liposomal vaccine adjuvant, Adamantylamide Dipeptide vaccine adjuvant, Aluminum vaccine adjuvant, Algal Glucan, Stearyl Tyrosine, Specol, Algammulin, Calcium Phosphate Gel, CTA1-DD gene fusion protein, DOC/Alum Complex, Gamma Inulin, Gerbu Adjuvant, GM-CSF, GMDP, Recombinant hlFN-gamma/Interferon-g, Interleukin- 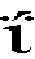 β, Interleukin-2, Interleukin-7, Sclavo peptide, Rehydragel LV, Rehydragel HPA, Loxoribine, MF59, MTP-PE Liposomes, Murametide, Murapalmitine, D-Murapalmitine, NAGO, Non-Ionic Surfactant Vesicles, PMMA, Protein Cochleates, QS-21, SPT (Antigen Formulation), nanoemulsion vaccine adjuvant, Quil-A vaccine adjuvant, RC529 vaccine adjuvant, LTR192G vaccine adjuvant, E. coli heat-labile toxin, LT, amorphous aluminum hydroxyphosphate sulfate adjuvant, Calcium phosphate vaccine adjuvant, Imiquimod, Resiquimod, Flagellin, Poly(LC), Albumin-heparin microparticles vaccine adjuvant, AS-2 vaccine adjuvant, B7-2 vaccine adjuvant, DHEA vaccine adjuvant, Immunoliposomes Containing Antibodies to Costimulatory Molecules, SAF-1, Sendai Proteo liposomes, Sendai-containing Lipid Matrices, Threonyl muramyl dipeptide (TMDP), Ty Particles vaccine adjuvant, Bupivacaine vaccine adjuvant, DL-PGL (Polyester poly (DL-lactide-co-glycolide)) vaccine adjuvant, IL-15 vaccine adjuvant, LTK72 vaccine adjuvant, MPL-SE vaccine adjuvant, non-toxic mutant El 12K of Cholera Toxin mCT-El 12K, Monophosphoryl lipid A (MPLA) and/or Matrix-S.

9. An immunomodulatory compound of any one of claims 1-4 for use as an adjuvant in vaccination.

10. An immunomodulatory compound of any one of claims 1-4 or an immunogenic composition of any one of claims 6-8 for use in the method of improving the immune response in a subject.

11. The immunomodulatory compound or the immunogenic composition for use of claim 10, wherein the subject has
a) an infectious disease caused by pathogenic microorganisms selected from the group comprising bacteria, viruses, fungi, protozoal pathogens and parasites.
b) a cancer, or
c) an autoimmune disease.

12. The immunomodulatory compound or the immunogenic composition for use of any one of claims 10-11, wherein the method further comprises administering to the subject one or more additional active agents selected from the group comprising
a) antibiotics, anti-bacterial agents, anti-viral agents, antifungal agents, antiparasitic agents, osmotic diuretics, anti-convulsants, anti-pyretics, immunomodulating drugs and/or combinations thereof, or
b) cancer immunotherapeutic agents selected from the group comprising immune checkpoint inhibitor, TCR-T cells, CAR-T cells and/or combinations thereof.

13. An immunogenic composition of any one of claims 6-8 or an immunomodulatory compound of any one of claims 1-4 for use in the method of treating and/or preventing an infectious disease, wherein the infectious disease is caused by pathogenic microorganisms selected from the group comprising bacteria, viruses, fungi, protozoal pathogens and parasites.

14. The immunogenic composition or the immunomodulatory compound for use of claim 13, wherein the method further comprises administering to the subject one or more additional active agents selected from the group comprising antibiotics, anti-bacterial agents, anti-viral agents, antifungal agents, antiparasitic agents, osmotic diuretics, anti-convulsants, anti-pyretics, immunomodulating drugs and combinations thereof.
